# EUROPEAN PATENT SPECIFICATION

(11) **EP 0 934 257 B1**
(45) Date of publication and mention of the grant of the patent: **11.12.2002**
(21) Application number: 97936050.0
(22) Date of filing: 16.07.1997
(51) Int. Cl.: C07C 255/58, A61K 31/275, C07C 225/20, C07C 233/41, A61K 31/16

(54) **SUBSTITUTED N-ARYLMETHYLAMINO DERIVATIVES OF CYCLOBUTENE-3,4-DIONES**
SUBSTITUIERTE N-ARYLMETHYLAMINO-CYCLOBUTEN-3,4-DION-DERIVATE
DERIVES A SUBSTITUTION N-ARYLMETHYLAMINO DE CYCLOBUTENE-3,4-DIONES

(30) Priority: 17.07.1996 US 678430; 17.07.1996 US 682207; 17.07.1996 US 684278; 17.07.1996 US 684279; 07.07.1997 US 889163; 07.07.1997 US 889164; 07.07.1997 US 889165; 07.07.1997 US 889166
(43) Date of publication of application: 11.08.1999
(60) Divisional application: 01119663.1
(73) Proprietor: Wyeth, Madison, New Jersey 07940-0874 (US)
(72) Inventor: ANTANE, Madelene, Miyoko, Lawrenceville, NJ 08648 (US); HERBST, David, Richard, Wayne, PA 19087 (US); McFARLANE, Geraldine, Ruth, Monmouth Junction, NJ 08852 (US); GUNDERSEN, Eric, Gould, Plainsboro, NJ 08536 (US); HIRTH, Bradford, Hammond, Littleton, MA 01460 (US); QUAGLIATO, Dominick, Anthony, Bridgewater, NJ 08807 (US); GRACEFFA, Russell, Francis, Plainsboro, NJ 08536 (US); BUTERA, John, Anthony, Clarksburg, NJ 08510 (US); GILBERT, Adam, Matthew, Rockland County, NY 10920 (US)
(74) Representative: Mannion, Sally Kim, Dr.
(86) International application number: US9711992
(87) International publication number: WO98002413

(56) References cited:
- EP-A- 0 099 122
- EP-A- 0 496 561
- US-A- 5 466 712
- PATENT ABSTRACTS OF JAPAN vol. 018, no. 361 (C-1222), 7 July 1994 & JP 06 092915 A (SUMITOMO METAL IND LTD), 5 April 1994, cited in the application

## Description

### Background of Invention

The present invention relates to novel 1, 2-diamino derivatives of cyclobutene 3,4-diones having pharmacological activity, to a process for their preparation, to pharmaceutical compositions containing them, and to their use, via potassium channel modulation, in the treatment of disorders associated with smooth muscle contraction. Such disorders include, but are not limited to, urinary incontinence, hypertension, asthma, premature labor, irritable bowel syndrome, congestive heart failure, angina and cerebral vascular disease.

Stemp et al. (EP-426379) disclose a class of amino substituted cyclobutenedione derivatives of chromans described as having blood pressure lowering activity and bronchodilatory activity . Takeno et al. (Public Patent Disclosure Bulletin No. 6-92915) report a series of diaminocyclobuten-3,4-diones . Our own efforts in this area have been disclosed in the following US Patents: 5,464,867, 5,466,712, 5,403,853, 5,403,854, 5,397,790, and 5,401,753. Several series of 1-amino-2-phenylalkylamino-cyclobutene-3,4-diones are reported as H-2 receptor antagonists by Algieri et al. in US Patent 4,390,701. Several related 1-amino-2-phenoxyalkylamino derivatives are disclosed by Nohara et al. in US Patent 4,673,747. Additionally, US Patent 5,240,946 and EP-496561 disclose diaminocyclobuten-3,4-diones useful as NMDA antagonists.

The syntheses of variously substituted 1,2-diamino-cyclobutene-3,4-diones are described in the following publications: Tietze et al., Chem Ber. 1991, 124, 1215; Tietze et al., Bioconjugate Chem. 1991, 2, 148; Ehrhardt et al., Chem. Ber. 1977, 110, 2506, Neuse et al., Liebigs Ann. Chem. 1973, 619, Ried et al., Liebigs Ann. Chem. 1973, 619, Kinney et al., J. Med. Chem. 1992, 35, 4702.

### Description of The Invention

Accordingly, the present invention discloses compounds represented by formula (I): 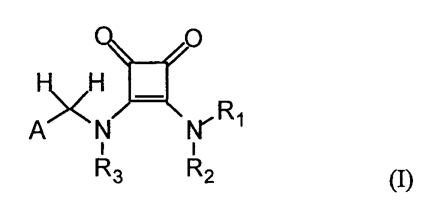 wherein:
R₁ is straight chain alkyl of 1 to 10 carbon atoms, branched chain alkyl of 3 to 10 carbon atoms, cycloalkyl of 3 to 10 carbon atoms, hydroxyalkyl of 2 to 10 carbon atoms or fluoroalkyl of 1 to 10 carbon atoms;
R₂ and R₃ are, independently, hydrogen or an acyl substituent selected from the group consisting of formyl, alkanoyl of 2 to 7 carbon atoms, alkenoyl of 3 to 7 carbon atoms, straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms, aralkoxycarbonyl of 6 to 12 carbon atoms, alkylsulfonyl of 1 to 7 carbon atoms, aroyl of 7 to 12 carbon atoms, arylalkenoyl of 9 to 20 carbon atoms, arylsulfonyl of 6 to 12 carbon atoms, arylalkanoyl of 8 to 12 carbon atoms or arylalkylsulfonyl of 7 to 12 carbon atoms; with the proviso that when R₃ is straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms or aralkoxycarbonyl of 6 to 12 carbon atoms, R₂ must be hydrogen;

A is a substituted phenyl group of the following formula: 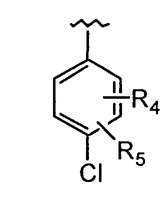 wherein:
R₄ and R₅ are, independently, cyano, nitro, amino, alkyl of 1 to 6 carbon atoms, fluoroalkyl of 1 to 6 carbon atoms, alkoxy of 1 to 6 carbon atoms, fluoroalkoxy of 1 to 6 carbon atoms, amino, alkylamino of 1 to 6 carbon atoms, dialkylamino of 2 to 12 carbon atoms, sulfamyl, alkylsulfonamido of 1 to 6 carbon atoms, arylsulfonamido of 6 to 12 carbon atoms, carbamoyl, alkylcarbamoyl of 2 to 7 carbon atoms, dialkylcarbamoyl of 4 to 14 carbon atoms, alkylcarboxamido containing 2 to 7 carbon atoms, arylcarboxamido containing 7 to 13 carbon atoms, alkylsulfonyl of 1 to 6 carbon atoms, perfluoroalkylsulfonyl of 1 to 6 carbon atoms, arylsulfonyl of 6 to 12 carbon atoms, chloro, bromo, fluoro, iodo, 1-imidazolyl, carboxyl or hydrogen, with the proviso that R₄ and R₅ cannot both be hydrogen; or a pharmaceutically acceptable salt thereof.

Another preferred aspect of this invention are those compounds of formula (I)
wherein A is 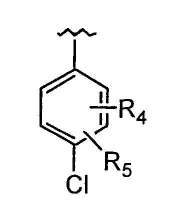
R₁ is straight chain alkyl of 1 to 10 carbon atoms, branched chain alkyl of 3 to 10 carbon atoms or fluoroalkyl of 1 to 10 carbon atoms;
R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen;
R₄ and R₅ are, independently, cyano, alkyl of 1 to 6 carbon atoms, fluoroalkyl of 1 to 6 carbon atoms, alkoxy of 1 to 6 carbon atoms, fluoroalkoxy of 1 to 6 carbon atoms, chloro, bromo, fluoro, iodo or hydrogen, with the proviso that R₄ and R₅ cannot both be hydrogen ;
or a pharmaceutically acceptable salt thereof.

A particularly preferred aspect of this invention includes compounds of formula (I) wherein A is : 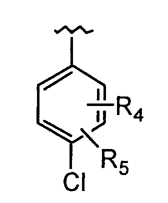
and R₁ is branched chain alkyl of 3 to 10 carbon atoms or fluoroalkyl of 1 to 10 carbon atoms;
R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen;
R₄ and R₅ are, independently, cyano, methyl, ethyl, trifluoromethyl, fluoroalkyl of 1 to 2 carbon atoms, methoxy, ethoxy, trifluoromethoxy, fluoroalkoxy of 1 to 2 carbon atoms, chloro, bromo, fluoro or hydrogen, with the proviso that R₄ and R₅ cannot both be hydrogen; or a pharmaceutically acceptable salt thereof.

When R₁ is fluoroalkyl it can be mono-, poly or per substituted fluoroalkyl. When R₄ and/or R₅ are fluoroalkyl or fluoroalkoxy, it can be mono-, poly or per susbsituted fluoroalkyl or fluoroalkoxy,

Preferred values of R₁ are straight chain alkyl of 1 to 10 carbon atoms, branched chain alkyl of 3 to 10 carbon atoms or fluoroalkyl of 1 to 10 carbon atoms. Particularly preferred values of R₁ are branched alkyl of 3 to 10 carbon atoms or fluoroalkyl of 1 to 10 carbon atoms.

Preferred values of R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen.

More preferred values of R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen.

Particularly preferred values of R₂ and R₃ are, independently, hydrogen, alkanoyl of 2 to 7 carbon atoms, alkenoyl of 3 to 7 carbon atoms, aroyl of 7 to 12 carbon atoms, or arylalkenoyl of 9 to 20 carbon atoms.

Preferred values of R₄ and R₅ are, independently, cyano, alkyl of 1 to 6 carbon atoms, fluoroalkyl of 1 to 6 carbon atoms, alkoxy of 1 to 6 carbon atoms, fluoroalkoxy of 1 to 6 carbon atoms, chloro, bromo, fluoro, iodo or hydrogen, with the proviso that R₄ and R₅ cannot both be hydrogen.

Particularly preferred values of R₄ and R₅ are, independently, cyano, methyl, ethyl, trifluoromethyl, fluoroalkyl of 1 to 2 carbon atoms, methoxy, ethoxy, trifluoromethoxy, fluoroalkoxy of 1 to 2 carbon atoms, chloro, bromo, fluoro or hydrogen, with the proviso that R₄ and R₅ cannot both be hydrogen;.

A preferred aspect of this invention involves compounds of formula (I) wherein R₁ is straight chain alkyl of 1 to 10 carbon atoms, branched chain alkyl of 3 to 10 carbon atoms, fluoroalkyl of 1 to 10 carbon atoms; R₂ and R₃ are, independently, hydrogen, alkanoyl of 2 to 7 carbon atoms, alkenoyl of 3 to 7 carbon atoms, aroyl of 7 to 12 carbon atoms, or arylalkenoyl of 9 to 20 carbon atoms.

A more preferred aspect of this invention are those compounds of formula (I) wherein R₁ is most preferably branched alkyl of 3 to 10 carbon atoms or fluoroalkyl of 1 to 10 carbon atoms; R₂ and R₃ are, independently, hydrogen, alkanoyl of 2 to 7 carbon atoms or alkenoyl of 3 to 7 carbon atoms.

Particularly preferred compounds of the invention are :
3-(2,4-dichlorobenzylamino)-4-(1,2,2-trimethylpropylamino)-cyclobut-3-ene-1,2-dione;
3-(2,4-dichloro-6-methyl-benzylamino)-4-(1,2,2-trimethyl-propylamino)-cyclobut-3-ene-1,2-dione;
3-(2,4-dichlorobenzylamino)-4-(1,1-dimethylpropylamino)-cyclobut-3-ene-1,2-dione;
N-(2,4-dichlorobenzyl)-N-[2-(1,1-dimethyl-propylamino)-3,4-dioxo-cyclobut-1-enyl]-acetamide;
N-(2,4-dichlorobenzyl)-N-[2-(1,1-dimethyl-propylamino)-3,4-dioxo-cyclobut-1-enyl]-butyramide;
3-(2,4-dichloro-6-methyl-benzylamino)-4-(1,1-dimethyl-propylamino)-cyclobut-3-ene-1,2-dione;
3-(t-butylamino)-4-(2,4-dichlorobenzylamino)-cyclobut-3-ene-1,2-dione;
3-tert-butylamino-4-(2,4-dichloro-6-methyl-benzylamino)-cyclobut-3-ene-1,2-dione;
3-(3,4-dichlorobenzylamino)-4-(1-ethyl-propylamino)-cyclobut-3-ene-1,2-dione;
3-(2,4-dichlorobenzylamino)-4-(1-ethyl-propylamino)-cyclobut-3-ene-1,2-dione;
3-(2,4-dichloro-6-methyl-benzylamino)-4-(2,2,3,3,3-pentafluoropropylamino)-cyclobut-3-ene-1,2-dione;
N-(2,4-dichloro-6-methyl-benzyl)-N-[2-(1,1-dimethyl-propylamino)-3,4-dioxo-cyclobut-1-enyl]-butyramide;
N-(2,4-dichloro-6-methyl-benzyl)-N-[2-(1,1-dimethyl-propylamino)-3,4-dioxo-cyclobut-1-enyl]-acetamide;
N-(tert-butyl)-N-[2-(2,4-dichloro-6-methyl-benzylamino)-3,4-dioxo-cyclobut-1-enyl]-propionamide;
3-(2,4-dichloro-6-methyl-benzylamino)-4-(1,2-dimethyl-2-fluoropropylamino)-cyclobut-3-ene-1,2-dione;
3-(2,4-dichlorobenzylamino)-4-(2-hydroxy-1,1-dimethylethylamino)-cyclobut-3-ene-1,2-dione;
(R)-3-(2,4-dichlorobenzylamino)-4-(1,2,2-trimethylpropylamino)-cyclobut-3-ene-1,2-dione;
3-tert-butylamino-4-(3,4-dichlorobenzylamino)-cyclobut-3-ene-1,2-dione;
3-(3,4-dichlorobenzylamino)-4-(1,1-dimethylpropylamino)-cyclobut-3-ene-1,2-dione;
(2,4-dichloro-6-methyl-benzyl)-[2-(1,1-dimethyl-propylamino)-3,4-dioxocyclobut-1-enyl]-carbamic acid tert-butyl ester;
or a pharmaceutically acceptable salts thereof.

It is understood that the definition of the compounds of formula (I), when R₁, R₂, R₃, R₄, or R₅ contain asymmetric carbons, encompass all possible stereoisomers and mixtures thereof which possess the activity discussed below. In particular, it encompasses racemic modifications and any optical isomers which possess the indicated activity. Optical isomers may be obtained in pure form by standard separation techniques. The pharmaceutically acceptable salts are those derived from such organic and inorganic acids as: lactic, citric, acetic, tartaric, succinic, maleic, malonic, hydrochloric, hydrobromic, phosphoric, nitric, sulfuric, methanesulfonic, and similarly known acceptable acids

The present invention also provides a process for the preparation of a compound of formula (I). More particularly, the compounds of formula (I) may be prepared by reacting a compound of formula (II): 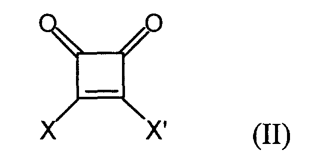 wherein X and X' is a suitably designed leaving group such as methoxy, ethoxy, butoxy, isopropoxy, halogeno, or a similar leaving group, with a compound of formula (III):

A₁―CH₂NH₂ (III)

wherein A₁ is A, as defined hereinbefore or a group of atoms convertible thereto, followed by treatment with a compound of formula (IV): 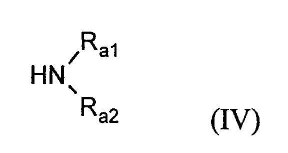 wherein Rₐ₁ and Rₐ₂ are R₁ and R₂, respectively, as defined hereinbefore or a group of atoms convertible thereto in a solvent such as ethanol, acetonitrile or the appropriate amine (IV) at elevated temperatures or room temperature. Dichloromethane can be used as a cosolvent. The order of addition of compound of formula (III) and compound of formula (IV) to compound of formula (II) may be reversed. Furthermore reaction of the sodium, potassium, or lithium salt of compound of formula (I) with the appropriate anhydride in tetrahydrofuran and/or N,N-dimethylformamide allows for the attachment of the acyl groups represented by R₃. Reaction of the sodium, potassium, or lithium salt of compound of formula (II), where X is a leaving group such as methoxy, ethoxy, butoxy, isopropoxy, or similar leaving group and X' is NHR₁, with the appropriate anhydride in dichloromethane, tetrahydrofuran and/or N,N-dimethylformamide or any other suitable solvent, followed by treatment with a compound of formula (III) as defined above in a solvent such as acetonitrile at room temperature allows for the attachment of the acyl groups represented by R₂.

Furthermore reaction of compound of formula (I) with the appropriate anhydride in pyridine with or without protection of the benzyl nitrogen allows for the attachment of R₂ and R₃.

As mentioned previously, the compounds of formula (I) have been found to relax smooth muscle. They are therefore useful in the treatment of disorders associated with smooth muscle contraction, disorders involving excessive smooth muscle contraction of the urinary tract (such as incontinence), or of the gastrointestinal tract (such as irritable bowel syndrome), asthma and hair loss. Furthermore, the compounds of formula (I) are active as potassium channel activators which render them useful for treatment of peripheral vascular disease, hypertension, congestive heart failure, stroke, anxiety, cerebral anoxia and other neurodegenerative disorders. Thus, the present compounds of formula (I) may be used in a method of treating smooth muscle disorders in mammals including man, which comprises administering to the afflicted mammal an effective amount of a compound or a pharmaceutical composition of the invention.

The present invention accordingly provides a pharmaceutical composition which comprises a compound of this invention in combination or association with a pharmaceutically acceptable carrier. In particular, the present invention provides a pharmaceutical composition which comprises an effective amount of a compound of this invention and a pharmaceutically acceptable carrier.

The compositions are preferably adapted for oral administration. However, they may be adapted for other modes of administration, for example, parenteral administration for patient suffering from heart failure.

In order to obtain consistency of administration, it is preferred that a composition of the invention is in the form of a unit dose. Suitable unit dose forms include tablets, capsules and powders in sachets or vials. Such unit dose forms may contain from 0.1 to 100 mg of a compound of the invention and preferably from 2 to 50 mg. Still further preferred unit dosage forms contain 5 to 25 mg of a compound of the present invention. The compounds of the present invention canbe administered orally at a dose range of about 0.01 to 100 mg/kg or preferably at a dose range of 0.1 to 10 mg/kg. Such compositions may be administered from 1 to 6 times a day, more usually from 1 to 4 times a day.

The compositions of the invention may be formulated with conventional excipients, such as a filler, a disintegrating agent, a binder, a lubricant, a flavoring agent and the like. They are formulated in conventional manner, for example, in a manner similar to that used for known antihypertensive agents, diuretics and β-blocking agents.

The following examples are presented to illustrate rather than limit the scope of the invention.

### EXAMPLE 1

### 3-Butoxy-4-(1,2,2-trimethyl-propylamino)-cyclobut-3-ene-1,2-dione

Tetrahydrofuran (15 mL), 3,4-dibutoxy-3-cyclobutene-1,2-dione (2.26 g, 10 mmol) and 2-amino-3,3-dimethylbutane (1.01 g, 10 mmol) were stirred together for approximately 65 hours at room temperature. The waxy solid remaining after removal of solvent was dissolved in approximately 15 mL chloroform and chromatographed (flash, ethyl acetate/hexane) on silica. The appropriate fractions were freed of solvent to yield 2.41 g (95%) of a cream-colored waxy solid: mp 90-9°C° (softens 85°C).

Two recrystallizations of 1.1 g of this material from hexane provided 0.833 g of the title compound as a white solid: mp 90-93C° (softens 88C°); ¹H NMR: (DMSO-d₆): δ 8.73 and 8.50 (two br d, 1H, rotamers), 4.64 (m, 2H), 3.92 and 3.41 (two m, 1H, rotamers), 1.71 (m, 2H), 1.38 (m, 2H), 1.11 (m, 3H), 0.91 (t, 3H), 0.84 (m, 9H) ppm. IR (KBr): 3135, 1800, 1690 cm⁻¹; MS (m/z): 253 (M⁺).

| Elemental Analysis for C₁₄H₂₃NO₃ | | | |
|---|---|---|---|
| Calcd | C, 66.37; | H, 9.15; | N, 5.53. |
| Found | C, 66.47; | H, 9.20; | N, 5.50. |

### EXAMPLE 2

### 3-(2,4-Dichlorobenzylamino)-4-(1,2,2-trimethylpropylamino)-cyclobut-3-ene-1,2-dione

Tetrahydrofuran (10 mL), 3-butoxy-4-(1,2,2-trimethyl-propylamino)-cyclobut-3-ene-1,2-dione (1.01 g, 3.99 mmol, Example 1) and 2,4-dichlorobenzylamine (0.70 g, 4.0 mmol) were stirred together at room temperature for 14 hours. Following removal of solvent, the residue was triturated with diethyl ether and dried. The off-white solid product was recrystallized twice from nitromethane to yield 0.408 g (29%) of the title compound as a white solid: mp 234-235°C; ¹H NMR: (DMSO-d₆): δ 7.67 (m, br, 1H), 7.65 (m, br, 1H), 7.48 (m, 2H), 7.32 (m, br, 1H), 4.80 (m, 2H), 3.90 (m, 1H), 1.10 (d, 3H), 0.86 (s, 9H) ppm. IR (KBr): 3140, 1790, 1640 cm⁻¹; MS (m/z): 354/356/358. HPLC indicates a major component (99%).

| Elemental Analysis for C₁₇H₂₀Cl₂N₂O₂ | | | |
|---|---|---|---|
| Calcd. | C, 57.47; | H, 5.67; | N, 7.88. |
| Found | C, 57.02; | H, 5.44; | N, 7.75. |
| | C, 57.69; | H, 5.69; | N, 7.79. |

### EXAMPLE 3

### 3-Ethoxy-4-(1,2,2-trimethyl-propylamino)-cyclobut-3-ene-1,2-dione

A solution of 3,4-diethoxy-3-cyclobutene-1,2-dione (10 g, 59 mmol) and (R)-2-amino-3,3-dimethylbutane (353 mL of a 0.2M solution in absolute ethanol, 71 mmol) was stirred at room temperature for 24 hours. Another portion of (R)-2-amino-3,3-dimethylbutane (150 mL of a 0.2 M solution in absolute ethanol, 30 mmol) was added and the resulting solution was stirred at room temperature for 24 hours. The slurry was filtered, and the filtrate concentrated under reduced pressure. The resulting solid was trituated with hexane:ethyl acetate (150:5 mL), then washed with hexane to give 9.78 g (74%) of (R)-3-ethoxy-4-(1,2,2-trimethyl-propylamino)-cyclobut-3-ene-1,2-dione as a white solid: ¹H NMR: (DMSO-d₆): δ 8.72 and 8.50 (two d, 1H, rotamers), 4.65 (m, 2H), 3.90 and 3.42 (two m, 1H, rotamers), 1.37 and 1.35 (two overlapping t, 3H, rotamers), 1.10 (two overlapping d, 3H, rotamers), 0.85 and 0.84 (two s, 9H, rotamers) ppm. IR (KBr): 3150, 2950, 1800, 1700 cm⁻¹; MS (m/z): 225 (M⁺).

| Elemental Analysis for C₁₂H₁₉NO₃ | | | |
|---|---|---|---|
| Calcd | C, 63.98; | H, 8.50; | N, 6.22. |
| Found | C, 64.33; | H, 8.54; | N, 6.52. |

(S)-3-Ethoxy-4-(1,2,2-trimethyl-propylamino)-cyclobut-3-ene-1,2-dione is produced by the same method by substituting (S)-2-amino-3,3-dimethylbutane for the (R)-2-amino-3,3-dimethylbutane employed in the preceding paragraph.

### EXAMPLE 4

### 3-(2,4-Dichloro-6-methyl-benzylamino)-4-(1,2,2-trimethylpropylamino)-cyclobut-3-ene-1,2-dione

The product of Example 3 (0.2 g, 0.88 mmol) and 2,4-dichloro-6-methylbenzylamine (0.17 g, 0.89 mmol, containing approximately 5% of a compound which is regioisomeric with respect to the substitution on the aryl ring) were placed in absolute ethanol (4.4 mL) and dichloromethane (2 mL). The resulting clear solution was allowed to stand at room temperature for 4 days. The reaction mixture was diluted with acetonitrile (5 mL) and filtered, rinsed with acetonitrile, and dried to give 0.3 g of a solid. Trituation with 10% methanol in dichloromethane gave 0.21 g (63%) of (R)-3-(2,4-dichloro-6-methyl-benzylamino)-4-(1,2,2-trimethyl-propylamino)-cyclobut-3-ene-1,2-dione as a white solid, which contains approximately 5% of a compound which is regioisomeric with respect to substitution on the aryl ring: mp >300°C; [α]²⁵_{D} = +31.86° (7.7 mg/mL, DMSO); ¹H NMR (DMSO-d₆) d 7.54 (d, 1H), 7.39 (d, 1H), 7.31 (m, 1H), 7.17 (m, 1H), 4.89 (m, 2H), 4.70 (doublet of m, minor isomer), 3.89 (m, 1H), 2.41 (s, 3H), 2.31 (s, minor isomer), 1.09 (d, 3H), 0.85 (s, 9H) ppm. IR (KBr): 3150,2950, 1800 cm ⁻¹; MS (m/z) 368/370/372 (M⁺).

| Elemental analysis for C₁₈H₂₂Cl₂N₂O₂ | | | |
|---|---|---|---|
| Calc'd | C, 58.54; | H, 6.01; | N, 7.59. |
| Found | C, 58.48; | H, 6.02; | N, 7.45. |

(S)-3-(2,4-Dichloro-6-methyl-benzylamino)-4-(1,2,2-trimethyl-propylamino)-cyclobut-3-ene-1,2-dione is produced by the same method by substituting (S)-3-ethoxy-4-(1,2,2-trimethyl-propylamino)-cyclobut-3-ene-1,2-dione for the (R)-3-ethoxy-4-(1,2,2-trimethyl-propylamino)-cyclobut-3-ene-1,2-dione employed in the preceding paragraph.

### EXAMPLE 5

### 3-Butoxy-4-(1,1-dimethyl-propylamino)-cyclobut-3-ene-1,2-dione

A solution of 3,4-dibutoxy-3-cyclobutene-1,2-dione (4.53 g, 20 mmol) and 1,1-dimethylpropylamine (1.74 g, 20 mmol) in tetrahydrofuran (20 mL) was stirred at room temperature for approximately 19.5 hours. The solvent was removed and the residue was chromatographed (gravity, chloroform/hexane) on neutral, activity III silica (150 g). The white solid isolated from the appropriate eluates was recrystallized from hexane to give 4.105 g (86%) of a white product: mp 56.5-57.5°C (softens 55.5°C).

One gram of this material was recrystallized twice from hexane to provide 0.794 g of the title compound as a white solid: mp 56-57°C (softens 55°C); ¹H NMR (DMSO-d₆): δ 8.63 and 8.48 (two br s, 1H, rotamers), 4.67 (m, br, 2H), 1.67 (m, br, 4H), 1.39 (m, 2H), 1.26 (m, br, 6H), 0.91 (t, 3H), 0.78 (t, 3H) ppm. IR (KBr): 3170, 1790, 1700 cm⁻¹; MS (m/z): 239 (M⁺).

| Elemental Analysis for C₁₃H₂₁NO₃ | | | |
|---|---|---|---|
| Calcd | C, 65.24; | H, 8.85; | N, 5.85 |
| Found | C, 65.12; | H, 8.90; | N, 5.77 |

### EXAMPLE 6

### 3-(2,4-Dichlorobenzylamino)-4-(1,1-dimethylpropylamino)-cyclobut-3-ene-1,2-dione

A solution of 3-butoxy-4-(1,1-dimethyl-propylamino)-cyclobut-3-ene-1,2-dione (7.18 g, 30 mmol, Example 5) and 2,4-dichlorobenzylamine (5.28 g, 30 mmol) in tetrahydrofuran (40 mL) was stirred at room temperature for 16 hours. The solvent was removed and the residue was triturated thoroughly with diethyl ether and dried to give 8.94 g of a crude product. Successive recrystallizations of this material from acetonitrile (charcoal), acetonitrile (twice), acetone (charcoal) and acetone (twice) afforded 4.08 g (40%) of the title compound as a white, electrostatic solid: mp 196-197°C (softens 188°C); ¹H NMR (DMSO-d₆): δ 7.81 (m, 1H), 7.68 (m, 1H), 7.48 (m, 3H), 4.81 (d, 2H), 1.67 (m, 2H), 1.31 (s, 6H), 0.82 (t, 3H) ppm. IR (KBr): 3210, 1790, 1645 cm⁻¹; MS (m/z) 340/342/344 (M⁺). HPLC indicates a major component (99% ). Differential scanning calorimetry studies indicate that this material is a mixture of crystal forms.

| Elemental Analysis for C₁₆H₁₈Cl₂N₂O₂ | | | |
|---|---|---|---|
| Calcd | C, 56.32; | H, 5.32; | N, 8.21. |
| Found | C, 55.93; | H, 5.20; | N, 8.18. |

### EXAMPLE 7

### N-(2,4-Dichlorobenzyl)-N-[2-(1,1-dimethyl-propylamino)-3,4-dioxo-cyclobut-1-enyl]-acetamide

To the product of Example 6 (0.60g, 1.26 mmol) in a mixture of tetrahydrofuran (8 mL) and dimethylformamide (2 mL) at ambient temperature under a nitrogen atmosphere was added NaH (0.077g of a 60% dispersion in mineral oil, 1.94 mmol). After stirring for 15 minutes at ambient temperature, acetic anhydride (0.183 mL, 1.94 mmol) was added neat. The mixture was stirred for 2 hours and was then diluted with brine and extracted with ethyl acetate (3 x 50 mL). The organic phase was washed with 10% aqueous Na₂CO₃ and brine, dried (MgSO₄), decolorized (charcoal), and concentrated to afford a residue. Crystallization from diethyl ether afforded 0.36 g (53%) of N-(2,4-dichlorobenzyl)-N-[2-(1,1-dimethylpropylamino)-3,4-dioxo-cyclobut-1-enyl]-acetamide as a white solid. mp: 114-116°C; ¹H NMR δ (DMSO-d₆) 7.66 (d, 1H), 7.32 (dd, 1H), 7.29 (d, 1H), 5.20 (br s, 2H), 2.15 (s, 3H), 1.70 (q, 2H), 1.35 (s, 6H), 0.85 (t, 3H) ppm. IR (KBr): 3400, 3300, 2950, 1800, 1700, 1580 cm⁻¹. MS (m/z) 382/384/386 (M⁺).

| Elemental analysis for C₁₈H₂₀Cl₂N₂O₃ | | | |
|---|---|---|---|
| Calc'd | C, 56.41; | H, 5.26; | N, 7.31. |
| Found | C, 56.30; | H, 5.27; | N, 7.25. |

### EXAMPLE 8

### N-(2,4-Dichlorobenzyl)-N-[2-(1,1-dimethyl-propylamino)-3,4-dioxo-cyclobut-1-enyl]-butyramide

To the product of Example 6 (0.60g, 1.26 mmol) in a mixture of tetrahydrofuran (8 mL) and dimethylformamide (2 mL) at ambient temperature under a nitrogen atmosphere was added NaH (0.077g of a 60% dispersion in mineral oil, 1.94 mmol). After stirring for 15 minutes at ambient temperature, butyric anhydride (0.317 mL, 1.94 mmol) was added neat. The mixture was stirred for 2 hours and was then diluted with brine and extracted with ethyl acetate (3 x 50 mL). The organic phase was washed with 10% aqueous Na₂CO₃ and brine, dried (MgSO₄), decolorized (charcoal), and concentrated to afford a clear oil. Crystallization from diethyl ether afforded 0.52 g (72%) of N-(2,4-dichlorobenzyl)-N-[2-(1,1-dimethylpropylamino)-3,4-dioxo-cyclobut-1-enyl]-butyramide as a white solid. mp: 112-117°C; ¹H NMR δ (DMSO-d₆) 7.65 (d, 1H), 7.43 (dd, 1H), 7.29 (d, 1H), 5.16 (br s, 2H), 2.42 (t, 2H), 1.72 (q, 2H), 1.54 (m, 2H), 1.35 (s, 6H), 0.83 (m, 6H) ppm. IR (KBr): 3400, 3300, 2950, 1800, 1725, 1580 cm⁻¹. MS (m/z) 410/412/414 (M⁺).

| Elemental analysis for C₂₀H₂₄Cl₂N₂O₃ | | | |
|---|---|---|---|
| Calc'd | C, 58.40; | H, 5.88; | N, 6.81. |
| Found | C, 58.40; | H, 5.84; | N, 6.86. |

### EXAMPLE 9

### 3-(2,4-Dichloro-6-methyl-benzylamino)-4-(1,1-dimethylpropylamino)-cyclobut-3-ene-1,2-dione

This compound was prepared in a procedure similar to the one described in Example 4. From 3-ethoxy-4-(1,1-dimethyl-propylamino)-cyclobut-3-ene-1,2-dione (16.67 g, 79.0 mmol) and 2,4-dichloro-6-methylbenzylamine (15.02 g, 79.0 mmol) in absolute ethanol (395 mL) there was obtained after filtration a white solid, which was washed with diethyl ether/hexane and dried in vacuo. This yielded 25.7 g (92%) of the title compound as a white solid: mp 247.1-248.3°C; ¹H NMR (DMSO-d₆) δ 7.54 (d, 1H), 7.44 (br t, 1H), 7.39 (d, 1H), 7.31 (s, 1H), 4.90 (d, 2H), 2.40 (s, 3H), 1.66 (q, 2H), 1.28 (s, 6H), 0.80 (t, 3H) ppm. IR (KBr): 3200, 2980, 1800, 1650 cm ⁻¹; MS (m/z) 354/356/358 (M⁺). Analytical HPLC indicates a major component (99.9%).

| Elemental analysis for C₁₇H₂₀Cl₂N₂O₂ | | | |
|---|---|---|---|
| Calc'd | C, 57.47; | H, 5.67; | N, 7.89. |
| Found | C, 57.31; | H, 5.50; | N, 7.80. |

### EXAMPLE 10

### 3-Butoxy-4-tert-butylamino-cyclobut-3-ene-1,2-dione

A solution of 3,4-dibutoxy-3-cyclobutene-1,2-dione (11.31 g, 50 mmol) and *tert*-butylamine (3.66 g, 50 mmol) in tetrahydrofuran (80 mL) was stirred at room temperature for 71 hours. The solvent was removed and a solution of the residue in chloroform was washed with water and dried (anhydrous Na₂SO₄). Removal of the solvent and chromatographic (gravity, chloroform/hexane) purification of the amber liquid residue on a column of neutral, activity III silica (350 g ) provided 9.83 g (87%) of a white solid product, mp 67.0-68.5°C. Two recrystallizations of an aliquot (800 mg) afforded 551 mg of the title compound as a white solid: mp 68-69°C (softens 67°C); ¹H NMR (DMSO-d₆): δ 8.75 and 8.59 (two br s, 1H, rotamers). 4.66 (m, br, 2H), 1.72 (m, 2H), 1.40 (m, 2H), 1.31 (m, 9H), 0.91 (t, 3H) ppm. IR (KBr): 3140, 1780, 1700 cm⁻¹; MS (m/z) 225 (M⁺).

| Elemental Analysis for C₁₂H₁₉NO₃ | | | |
|---|---|---|---|
| Calcd | C, 63.98; | H, 8.50; | N, 6.22. |
| Found | C, 64.13; | H, 8.60; | N, 6.24. |

### EXAMPLE 11

### 3-(t-Butylamino)-4-(2,4-dichlorobenzylamino)-cyclobut-3-ene-1,2-dione

A solution of 3-butoxy-4-tert-butylamino-cyclobut-3-ene-1,2-dione (1.13 g, 5.0 mmol, Example 10) and 2,4-dichlorobenzylamine (0.884 g, 5.0 mmol) in tetrahydrofuran (15 mL ) was stirred at room temperature for 16.5 hours. Removal of solvent, thorough trituration of the residue with diethyl ether and drying gave 1.50 g of a solid. Two recrystallizations of the crude product from acetonitrile afforded 1.22 g (74%) of the title compound as a white solid: mp 229-230°C (dec.); ¹H NMR (DMSO-d₆): δ 7.77 (m, 1H), 7.68 (m, 1H), 7.61 (s, br, 1H), 7.48 (m, 2H), 4.79 (d, 2H), 1.36 (s, 9H) ppm. IR (KBr): 3300, 3220, 1780, 1660 cm⁻¹; MS (m/z): 326/328/330 (M⁺). HPLC indicates a major component (99.6%).

| Elemental Analysis for C₁₅H₁₆Cl₂N₂O₂ | | | |
|---|---|---|---|
| Calcd | C, 55.06; | H, 4.93; | N, 8.56 |
| Found | C, 54.86; | H, 4.89; | N, 8.48 |

### EXAMPLE 12

### 3-tert-Butylamino-4-(2,4-dichloro-6-methyl-benzylamino)-cyclobut-3-ene-1,2-dione

This compound was prepared in a procedure similar to the one described in Example 4. From 3-ethoxy-4-tert-butylamino-cyclobut-3-ene-1,2-dione (0.22 g, 1.1 mmol) and 2,4-dichloro-6-methylbenzylamine (0.22 g, 1.2 mmol, containing approximately 5% of a compound which is regioisomeric with respect to the substitution on the aryl ring) in absolute ethanol (5.5 mL) there was obtained 0.34 g (89%) of 3-*tert*-butylamino-4-(2,4-dichloro-6-methyl-benzylamino)- cyclobut-3-ene-1,2-dione as a white solid, which contains approximately 5% of a compound which is regioisomeric with respect to substitution on the aryl ring: mp 264-268°C; ¹H NMR (DMSO-d₆) δ 7.54 (d, 1H), 7.46 (s, 1H), 7.43 (br t, 1H), 7.39 (d, 1H), 4.89 (d, 2H), 4.72 (d, minor isomer), 2.40 (s, 3H), 2.31 (s, minor isomer), 1.34 (s, 9H) ppm. IR (KBr): 3200, 2950, 1800, 1650 cm ⁻¹; MS (m/z) 340/342/344 (M⁺).

| Elemental analysis for C₁₆H₁₈Cl₂N₂O₂ | | | |
|---|---|---|---|
| Calc'd | C, 56.32; | H, 5.32; | N, 8.21. |
| Found | C, 56.09; | H, 5.28; | N, 8.16. |

### EXAMPLE 13

### 3-Butoxy-4-(1-ethyl-propylamino)-cyclobut-3-ene-1,2-dione

A solution of 3,4-dibutoxy-3-cyclobut-3-ene-1,2-dione (2.26 g, 10 mmol) and 1-ethylpropylamine (0.872 g, 10 mmol) in tetrahydrofuran (8 mL ) was stirred at room temperature for 2.5 hours. The residue remaining after removal of solvent was dissolved in chloroform and the solution was washed with water and dried (anhydrous Na₂SO₄). Removal of solvent gave a waxy solid that was chromatographed (flash, chloroform/hexane) on silica. The solid isolated from the appropriate fractions was recrystallized twice from hexane to yield 0.896 g (37%) of the title compound: mp 65-66°C; ¹H NMR (DMSO-d₆): δ 8.63 and 8.40 (two d, 1H, rotamers), 4.64 (m, 2H), 3.74 and 3.30 (two m, 1H, rotamers), 1.71 (m, 2H), 1.54 (m, 2H), 1.39 (m, 4H), 0.90 (m, 3H), 0.82 (m, 6H) ppm. IR (KBr): 3140, 1790, 1720 cm⁻¹; MS (m/z) 239 (M⁺).

| Elemental Analysis for C₁₃H₂₁NO₃ | | | |
|---|---|---|---|
| Calcd | C, 65.25; | H, 8.85; | N, 5.85. |
| Found | C, 65.37; | H, 9.07; | N, 5.87. |

### EXAMPLE 14

### 3-(3,4-Dichlorobenzylamino)-4-(1-ethyl-propylamino)-cyclobut-3-ene-1,2-dione

This compound was prepared in a manner similar to Example 6 using the appropriate starting materials, to afford 3-(3,4-dichlorobenzylamino)-4-(1-ethylpropylamino)- cyclobut-3-ene-1,2-dione as a white solid: mp 268-269°C.

### EXAMPLE 15

### 3-(2,4-Dichlorobenzylamino)-4-(1-ethyl-propylamino)-cyclobut-3-ene-1,2-dione

This compound was prepared in a manner similar to Example 6 using the appropriate starting materials to afford 3-(2,4-dichlorobenzylamino)-4-(1-ethylpropylamino)-cyclobut-3-ene-1,2-dione as very pale yellow solid: mp 210-211°C.

### EXAMPLE 16

### 3-(2,4-Dichloro-6-methyl-benzylamino)-4- (2,2,3,3,3-pentafluoropropylamino)-cyclobut-3-ene-1,2-dione

A solution of 3,4-diethoxy-3-cyclobutene-1,2-dione (4.2 mL, 28.4 mmol) and 2,2,3,3,3-pentafluoropropylamine (4.24 g, 28.4 mmol) in absolute ethanol (142 mL) was stirred at room temperature for 24 hours. The solution was concentrated under reduced pressure and triturated with 10% ethyl acetate in hexane to yield 1.14 g (14.6%) of a white solid,: mp 95-100°C; ¹H NMR (DMSO-d₆) δ 9.40 and 9.20 (two br m, 1H, rotamers), 4.67 (q, 2H), 4.33 and 4.11 (two br t, 2H, rotamers), 1.36 (br m, 3H); MS (m/z) 274 ([M+H]⁺). Following the procedure described in Example 4, from a portion of this solid, 3-ethoxy-4-(2,2,3,3,3-pentafluoro-propylamino)-cyclobut-3-ene-1,2-dione (0.72 g, 2.6 mmol) and 2,4-dichloro-6-methylbenzylamine (0.5 g, 2.6 mmol) there was obtained 1.03 g (94%) of the title compound as a white solid: mp 287-292°C; ¹H NMR (DMSO-d₆) δ 7.62 (br m, 2H), 7.53 (s, 1H), 7.38 (d, 1H), 4.89 (d, 2H), 4.43 (doublet of t, 2H), 2.40 (s, 3H) ppm.

| Elemental analysis for C₁₅H₁₁Cl₂ F₅N₂O₂ | | | |
|---|---|---|---|
| Calc'd | C, 43.19; | H, 2.66; | N, 6.72. |
| Found | C, 43.13; | H, 2.61; | N, 6.74. |

### EXAMPLE 17

### N-(2,4-Dichloro-6-methyl-benzyl)-N-[2-(1,1-dimethyl-propylamino)-3,4-dioxo-cyclobut-1-enyl]-butyramide

To 3-(2,4-dichloro-6-methyl-benzylamino)-4-(1,1-dimethyl-propylamino)-cyclobut-3-ene-1,2-dione (0.50 g, 1.41 mmol) in N,N-dimethylformamide (2 mL) and tetrahydrofuran (8 mL) was added sodium hydride (0.062 g of a 60% dispersion in mineral oil, 1.54 mmol) at 0°C. The frothy suspension was stirred for 1 hour as the mixture was warmed to 25°C. Butyric anhydride (0.24 g, 1.54 mmol) was added and the reaction mixture was stirred at 0°C for 15 minutes and then allowed to warm to room temperature. After stirring overnight, the reaction mixture was poured into brine (50 mL) and extracted with ethyl acetate (3 x 50 mL). The organic layer was dried over magnesium sulfate and decolorized (charcoal). The solvent was removed *in vacuo* and the remaining oil was triturated with diethyl ether/petroleum ether to yield 0.31 g (53%) of a white solid: mp 117.2-118.4°C; ¹H NMR (DMSO-d₆) δ 8.80 (br s, 1H), 7.39 (d, 1H), 7.28 (d, 1H), 5.06 (s, 2H), 2.34 (s, 3H), 2.29 (t, 2H), 1.67 (q, 2H), 1.51 (q, 2H), 1.30 (s, 6H), 0.82 (q, 6H) ppm. IR (KBr): 3230, 2950, 1800, 1744, 1700, 1570 cm⁻¹; MS (m/z) 424 (M⁺).

| Elemental analysis for C₂₁H₂₆Cl₂N₂O₃ | | | |
|---|---|---|---|
| Calc'd | C, 59.30; | H, 6.16; | N, 6.59. |
| Found | C, 59.34; | H, 6.09; | N, 6.52. |

### EXAMPLE 18

### N-(2,4-Dichloro-6-methyl-benzyl)-N-[2-(1,1-dimethyl-propylamino)-3,4-dioxo-cyclobut-1-enyl]-acetamide

This compound was prepared according to the procedure described in Example 17. From 3-(2,4-dichloro-6-methyl-benzylamino)-4-(1,1-dimethylpropylamino)-cyclobut-3-ene-1,2-dione (0.50 g, 1.41 mmol) and acetic anhydride (0.16 g, 1.54 mmol) there was obtained 0.36 g (64%) of the title compound as a white solid: mp 112.2-113.9°C; ¹H NMR (DMSO-d₆) δ 8.71 (br s, 1H), 7.40 (d, 1H), 7.29 (d, 1H), 5.06 (s, 2H), 2.35 (s, 2H), 2.05 (s, 3H), 1.67 (q, 2H), 1.30 (s, 6H), 0.81 (t, 3H) ppm. IR (KBr): 3230, 2950, 1800, 1755, 1590 cm⁻¹; MS (m/z) 396 (M⁺).

| Elemental analysis for C₁₉H₂₂Cl₂ N₂O₃ | | | |
|---|---|---|---|
| Calc'd | C, 57.44; | H, 5.58; | N, 7.05. |
| Found | C, 57.16; | H, 5.52; | N, 6.94. |

### EXAMPLE 19

### N-(tert-butyl)-N-[2-(2,4-Dichloro-6-methyl-benzylamino)-3,4-dioxo-cyclobut-1-enyl]-propionamide

To 3-butoxy-4-tert-butylamino-cyclobut-3-ene-1,2-dione (0.50 g, 2.5 mmol) in tetrahydrofuran (12.6 mL) was added sodium hydride (0.091 g of a 80% dispersion in mineral oil, 3.0 mmol). The suspension was stirred for 20 minutes at room temperature. The slightly cloudy yellow solution was concentrated under reduced pressure, and the resulting white solid was suspended in propionic anhydride (2 mL) and dichloromethane (3 mL). The reaction mixture was stirred at room temperature for 18 hours, then heated at 115°C for 24 hours. After standing at room temperature for 5 days the reaction mixture was diluted with dichloromethane, filtered, and concentrated under reduced pressure. Purification by column chromatography (silica gel, hexane/ethyl acetate) gave 0.09 g of material that was placed in acetonitrile (1.8 mL). 2,4-Dichloro-6-methylbenzylamine (67 mg, 0.35 mmol) and acetonitrile (2 mL) was added. After 3 days at room temperature, the reaction mixture was concentrated under reduced pressure, and the resulting solid was recrystallized from hexane and ethyl acetate to give 74 mg (53%) of a white solid: mp 177-180°C; ¹H NMR (DMSO-d₆) δ 9.31 (t, 1H), 7.54 (d, 1H), 7.40 (d, 1H), 4.94 (d, 2H), 2.40 (s, 3H), 2.09 (q, 2H), 1.37 (s, 9H), 0.87 (t, 3H) ppm.

| Elemental analysis for C₁₉H₂₂Cl₂N₂O₃ | | | |
|---|---|---|---|
| Calc'd | C, 57.44; | H, 5.58; | N, 7.05. |
| Found | C, 56.41; | H, 5.19; | N, 6.98. |

### EXAMPLE 20

### 3-(2,4-dichloro-6-methyl-benzylamino)-4-(1,2-dimethyl-2-fluoro-propylamino)-cyclobut-3-ene-1,2-dione

### Step 1) 3-Fluorovalinol

To a solution of lithium borohydride (1.61 g, 74 mmol) in THF(40 mL) under a nitrogen atmosphere was added trimethylsilyl chloride(18.8 mL, 148 mmol) via pipet. A precipitate quickly formed. After 3 minutes, 3-fluorovaline (5 g, 37 mmol) was added in three portions. This mixture was stirred for 24 hours. The reaction was quenched by the dropwise addition of methanol. The methanol and THF were removed on a rotary evaporator (30 degree water bath) and water (25 mL) was added. The aqueous mixture was made basic with 2.5N aq. NaOH and was then extracted with dichloromethane (4 x 50 mL). The combined organics were dried(Na₂SO₄), filtered and evaporated to give 3.83 g of 3-fluorovalinol: ¹H NMR (CDCl₃) δ 3.71 (dd, 1H), 3.36 (m, 1H), 2.90 (m,1H), 2.10 (br, 2H), 1.38 (d, 3H), 1.33 (d, 3H) ppm.

### Step 2) N-Butoxycarbonyl-3-fluorovalinol

To a solution of 3-fluorovalinol (3.79 g, 31.4 mmol) in chloroform (35 mL) under a nitrogen atmosphere was added a solution of di-t-butyl dicarbonate (6.84 g, 31.4 mmol) in chloroform (15 mL). The mixture was stirred at room temperature for four hours, then the solvent was removed on a rotary evaporator. The residue was dissolved in diethyl ether (100 mL), washed with 20% phosphoric acid (1 x50 mL), brine (1 x 50 mL), saturated aqueous sodium bicarbonate (1 x 50 mL), brine (1 x 50 mL), and then dried(MgSO₄). Filteration and concentration under reduced pressure gave 6.34 g of N-butoxycarbonyl-3-fluorovalinol as a white solid: ¹H NMR (CDCl₃) δ 5.08 (br, 1H), 3.82 (m, 2H), 3.68 (m, 1H), 1.46 (s, 9H), 1.46 (d, 3H) and 1.39 (d, 3H) ppm.

### Step 3) N-Butoxycarbonyl-1-iodo-2-amino-3-fluoro-3-methyl-n-butane

To a well-stirred mixture of polystyryl supported triphenyl phosphine (29.3 mmol) in dry dichloromethane (40 mL) under a nitrogen atmosphere was added iodine (7.44 g, 29.3 mmol). After ten minutes, imidazole (2.0 g, 29.3 mmol) was added followed in ten minutes by a solution of N-butoxycarbonyl-3-fluorovalinol (13.3 mmol) in dichloromethane (200 mL). The mixture was heated to reflux for two hours. The cooled mixture was filtered through Celite® and the filtrate was evaporated. The residue was dissolved in diethyl ether (150 mL) and this solution was washed with dilute aqueous sodium thiosulfate (1 x 75 mL) and water (2 x 75 mL). The organic layer was dried (Na₂SO₄), filtered through a pad of silica gel and evaporated to afford 3.46 grams of N-butoxycarbonyl-1-iodo-2-amino-3-fluoro-3-methyl-n-butane: ¹H NMR (CDCl₃) δ 4.72 (br d, 1H), 3.86 (br m, 1H), 3.56 (dd, 1H), 1.47 (s, 9H), 1.43 (m, 6H) ppm.

### Step 4) N-Butoxycarbonyl-2-amino-3-fluoro-3-methyl-n-butane

A Parr bottle was charged with palladium (II) hydroxide (800 mg), a solution of N-butoxycarbonyl-1 -iodo-2-amino-3-fluoro-3-methyl-n-butane (3.26 g, 9.8 mmol) in ethanol (80 mL) and triethylamine (0.99 g, 9.8 mmol). The reaction mixture was placed under hydrogen gas (50 psig) and shaken for 20 hours. The mixture was filtered through celite and evaporated. The residue was dissolved in diethyl ether (100 mL) and washed with 1N aq. HCl (2 x 50 mL), water (2 x 50 mL), and then dried (MgSO₄). Filtration and evaporation gave a residue that was chromatographed (silica gel, diethyl ether/hexane (3/1)) to afford 1.80 g of N-butoxycarbonyl-2-amino-3-fluoro-3-methyl-n-butane: ¹H NMR (CDCl₃) δ 4.65 (br, 1H), 3.70 (br m, 1H), 1.45 (s, 9H), 1.39 (d, 3H), 1.32 (d, 3H) and 1.18 (d, 3H) ppm.

### Step 5) 3-Ethoxy-4-(3-fluoro-3-methyl-n-butyl-2-amino)-3-cyclobutene-1,2-dione

A mixture of N-butoxycarbonyl-2-amino-3-fluoro-3-methyl-n-butane (1.75 g, 8.5 mmol), dichloromethane (5 mL), trifluoroacetic acid (4 mL), and methanol (0.75 mL) was warmed to 45°C for five hours. The volatile components were removed on a rotary evaporator and the syrupy residue was used without further purification. To a solution of 3-fluoro-3-methyl-n-butyl-2-amine trifluoroacetate salt (8.5 mmol) in ethanol (42.5 mL) was added 3,4-diethoxy-3-cyclobutene-1,2-dione (1.44 g, 8.5 mmol) followed by triethylamine (2.58 g, 25.5 mmol). The reaction mixture was stirred under a nitrogen atmosphere at room temperature for two hours then the temperature was raised to 50°C for five hours. The mixture was cooled and the solvents removed on a rotary evaporator. The residue was dissolved in diethyl ether (90 mL) and washed with water (1 x 60 mL), 1 N aq. HCl (1 x 60 mL), water 1 x 60 mL). The organic layer was dried (MgSO₄), filtered, and evaporated. The residue was chromatographed (silica gel, diethyl ether) to afford 1.65 g of 3-ethoxy-4-(3-fluoro-3-methyl-n-butyl-2-amino)-3-cyclobutene-1,2-dione as a white solid: ¹H NMR (CDCl₃) δ 6.21 (br, 1H), 4.77 (br m, 2H), 3.80 (br, 1H), 1.47 (t, 3H), 1.43 (d, 3H), 1.36 (d, 3H) and 1.32 (d, 3H) ppm.

### Step 6) 3-(2,4-dichloro-6-methyl-benzylamino)-4-(1,2-dimethyl-2-fluoropropylamino)-cyclobut-3-ene-1,2-dione

To a solution of 3-ethoxy-4-(3-fluoro-3-methyl-n-butyl-2-amino)-3-cyclobutene-1,2-dione (0.573 g, 2.5 mmol) in dry THF (8 mL) was added 2,4-dichloro-6-methylbenzylamine (0.523 g, 2.75 mmol). The mixture was heated to 70°C under a nitrogen atmosphere for 18 hours. The mixture was cooled to room temperature with stirring and vacuum filtered through a fritted glass filter. The solid was washed well with several portions of an ethanol/diethyl ether (1/1) solvent mixture. The solid was air dried then heated to 77°C under high vacuum for 16 hours. This afforded 0.48 g of the title compound as a white solid: ¹H NMR (DMSO-d₆) δ 7.54 (s, 1H), 7.40 (br, 1H), 7.38 (s, 2H), 4.90 (m, 2H), 4.17 (br, 1H), 2.41 (s, 3H), 1.32 (d, 3H), 1.27 (d, 3H) ,1.18 (d, 3H) ppm. IR (KBr): 1850 cm⁻¹; MS (m/z) 373 ([M+H]⁺).

### EXAMPLE 21

### 3-Butoxy-4-(2,4-dichlorobenzylamino)-cyclobut-3-ene-1,2-dione

A solution of 3,4-dibutoxy-3-cyclobutene-1,2-dione (3.39 g, 15 mmol) and 2,4-dichlorobenzylamine (2.64 g, 15 mmol) in tetrahydrofuran (20 mL) was stirred at room temperature for 5.5 hours. After removal of solvent, the residue was dissolved in chloroform (approximately 30 mL) and chromatographed (flash, ethyl acetate/hexane) on silica. The appropriate fractions were freed of solvent to give 4.31 g (88%) of a white product: mp 140-142°C (softens 137°C). Three recrystallizations of 1.1 g of this material from methyl *t*-butyl ether provided 0.566 g of the title compound as a white solid: mp 139.5-140.0°C (softens 137.5°C; ¹H NMR (DMSO-d₆) δ 9.25 and 9.00 (two m, 1H, rotamers), 7.64 (d, 1H), 7.51-7.34 (m, 2H), 4.69 (m, 2H), 4.56 (s, 2H), 1.72 (m, 1H), 1.58 (m, 1H), 1.38 (m, 1H), 1.19 (m, 1H), 0.91 and 0.82 (two t, 3H, rotamers) ppm. IR (KBr): 3160, 1760, 1700 cm⁻¹. MS (m/z) 327/329/331 (M⁺). HPLC indicates a major component (>99%).

| Elemental analysis for C₁₅H₁₅Cl₂NO₃ | | | |
|---|---|---|---|
| Calc'd | C, 54.90; | H, 4.61; | N, 4.27. |
| Found | C, 54.98; | H, 4.51; | N, 4.11. |

### EXAMPLE 22

### 3-(2,4-Dichlorobenzylamino)-4-(2-hydroxy-1,1-dimethylethylamino)-cyclobut-3-ene-1,2-dione

Tetrahydrofuran (10 mL), 3-butoxy-4-(2,4-dichlorobenzylamino)-cyclobut-3-ene-1,2-dione (1.31 g, 3.94 mmol, Example 21), and 2-amino-2-methyl-1-propanol (0.36 g, 4.0 mmol) were stirred together for 43.5 hours at room temperature. Following removal of solvent, the residue was trituated with diethyl ether and dried to yield 1.13 g of a yellow solid. Three recrystallizations of the crude product from methanol gave 0.503 g of the title compound as cream-colored solid: mp 237-238°C (softens 234°C); ¹H NMR (DMSO-d₆) δ 7.94 (t, 1H), 7.68 (m, 1H), 7.54 (m, 1H), 7.48 (m, 2H), 5.04 (t, 1H), 4.79 (d, 2H), 3.39 (d, 2H), 1.15 (s, 6H) ppm. IR (KBr): 3380, 3250, 1780, 1650 cm⁻¹. MS (m/z) 343 ([M+H]⁺). HPLC indicates a major component (99.9%).

| Elemental analysis for C₁₅H₁₆Cl₂N₂O₃ | | | |
|---|---|---|---|
| Calc'd | C, 52.49; | H, 4.70; | N, 8.16. |
| Found | C, 52.57; | H, 4.59; | N, 8.12. |

### EXAMPLE 23

### 3-(2,4-Dichlorobenzylamino)-4-(1,2,2-trimethylpropylamino)-cyclobut-3-ene-1,2-dione

The product of Example 3 (0.901 g, 4.0 mmol) and 2,4-dichlorobenzylamine (0.704 g, 4.0 mmol) in tetrahydrofuran (20 mL) were stirred at room temperature for approximately 16 hours and then were refluxed for approximately 24 hours. After removal of solvent, the residue was recrystallized from methanol (charcoal) and again recrystallized from methanol to afford 0.25 g (18%) of (R)-3-(2,4-dichlorobenzylamino)-4-(1,2,2-trimethylpropylamino)-cyclobut-3-ene-1,2-dione as a white solid: mp 235-239°C (softens 233°C); [α]²⁵_{D} = +26.28° (9.58 mg/mL, DMSO); ¹H NMR (DMSO-d₆) δ 7.67 (s, 1H), 7.63 (br s, 1H), 7.48 (m, 2H), 7.30 and 7.15 (two br d, 1H, rotamers), 4.80 (m, 2H), 3.90 (br s, 1H), 1.10 (m, 3H), 0.86 (m, 9H) ppm. IR (KBr): 3180, 1800, 1650 cm⁻¹. MS (m/z) 354/356/358 (M⁺). Analytical HPLC indicates chemical purity (96%) and optical purity (100%).

| Elemental analysis for C₁₇H₂₀Cl₂N₂O₃ | | | |
|---|---|---|---|
| Calc'd | C, 57.48; | H, 5.68; | N, 7.89. |
| Found | C, 57.96; | H, 5.86; | N, 7.77. |
| | C, 58.11; | H, 5.76; | N, 8.05. |

(S)-3-(2,4-Dichlorobenzylamino)-4-(1,2,2-trimethylpropylamino)-cyclobut-3-ene-1,2-dione is produced by the same method by substituting (S)-3-ethoxy-4-(1,2,2-trimethyl-propylamino)-cyclobut-3-ene-1,2-dione for the (R)-3-ethoxy-4-(1,2,2-trimethyl-propylamino)-cyclobut-3-ene-1,2-dione employed in the preceding paragraph.

### EXAMPLE 24

### 3-tert-Butylamino-4-(3,4-dichlorobenzylamino)-cyclobut-3-ene-1,2-dione

A solution of 3-butoxy-4-tert-butylamino-cyclobut-3-ene-1,2-dione (1.13 g, 5 mmol, Example 10) and 3,4-dichlorobenzylamine (0.880 g, 5.0 mmol) in tetrahydrofuran (15 mL) was stirred at room temperature for approximately 96 hours. The residue isolated after removal of solvent was recrystallized from N,N-dimethylformamide (twice) and from 2-methoxyethanol to provide 0.687 g (42%) of the title compound as a white solid: mp 302-303°C; ¹H NMR (DMSO-d₆) δ 7.77 (m, 1H), 7.65 (d, 1H), 7.62 (d, 1H), 7.55 (s, 1H), 7.33 (m, 1H), 4.71 (d, 2H), 1.35 (s, 9H) ppm. IR (KBr): 3450, 3230, 1800, 1650 cm⁻¹. MS (m/z) 326/328/330 (M⁺). HPLC indicates a major component (>99%).

| Elemental analysis for C₁₅H₁₆Cl₂N₂O₂ | | | |
|---|---|---|---|
| Calc'd | C, 55.06; | H, 4.93; | N, 8.56. |
| Found | C, 54.81; | H, 4.56; | N, 8.44. |

### EXAMPLE 25

### 3-(3,4-Dichlorobenzylamino)-4-(1,1-dimethylpropylamino)-cyclobut-3-ene-1,2-dione

Tetrahydrofuran (15 mL), 3-butoxy-4-(1,1-dimethylpropylamino)-cyclobut-3-ene-1,2-dione (1.20 g, 5.0 mmol) and 3,4-dichlorobenzylamine (0.88 g, 5.0 mmol) were stirred together at room temperature for approximately 16 hours. Removal of solvent gave 1.67 g of a white solid which was recrystallized from methanol (twice) to yield 1.06 g (62%) of the title compound as a white solid: mp 277-279°C; ¹H NMR (DMSO-d₆) δ 7.80 (m, 1H), 7.65 (d, 1H), 7.62 (d, 1H), 7.42 (s, 1H), 7.34 (m, 1H), 4.73 (d, 2H), 1.66 (m, 2H), 1.30 (s, 6H), 0.81 (t, 3H) ppm. IR (KBr): 3300, 3240, 1790, 1650 cm⁻¹. MS (m/z) 340/342/344 (M⁺). HPLC indicates a major component (98.9%).

| Elemental analysis for C₁₆H₁₈Cl₂N₂O₂ | | | |
|---|---|---|---|
| Calc'd | C, 56.32; | H, 5.32; | N, 8.21. |
| Found | C, 56.34; | H, 5.03; | N, 8.03. |

The smooth muscle relaxing activity of the compounds of this invention was established in accordance with standard pharmacologically accepted test procedures with representative compounds as follows:

Sprague-Dawley rats (150-200 g) are rendered unconscious by CO₂ asphyxiation and then euthanized by cervical dislocation. The bladder is removed into warm (37 deg.C) physiological salt solution (PSS) of the following composition (mM): NaCl, 118.4; KCl, 4.7; CaCl₂, 2.5; MgSO₄, 4.7; H₂O, 1.2; NaHCO₃, 24.9; KH₂PO₄, 1.2; glucose, 11.1; EDTA, 0.023; gassed with 95% O₂; 2/5% CO₂; pH 7.4. The bladder is opened and then cut into strips 1-2 mm in width and 7-10 mm in length. The strips are subsequently suspended in a 10 mL tissue bath under an initial resting tension of 1.5 g. The strips are held in place by two surgical clips, one of which is attached to a fixed hook while the other is attached to an isometric force transducer. The preparations, which usually exhibit small spontaneous contractions, are allowed to recover for a period of 1 hour prior to a challenge with 0.1 µM carbachol. The carbachol is then washed out and the tissue allowed to relax to its resting level of activity. Following a further 30 minute period of recovery, an additional 15 mM of KCl is introduced into the tissue bath. This increase in KCl concentration results in a large increase in the amplitude of spontaneous contractions (and initiation of contractions in previously quiescent strips) superimposed upon a small increase in basal tone. Following stabilization of this enhanced level of contractile activity, incremental increases in the concentration of test compound or vehicle are introduced into the tissue bath. Contractile activity is measured for each compound or vehicle concentration during the last minute of a 30 minute challenge.

The isometric force developed by the bladder strips is measured using a concentration required to elicit 50% inhibition of pre-drug contractile activity (IC₅₀ concentration) and is calculated from this concentration-response curve. The maximum percentage inhibition of contractile activity evoked by a test compound is also recorded for concentrations of test compound less than or equal to 30 µM.

The results of this study are shown in Table I.

**Table I**

| Inhibition of Contractions in Isolated Rat Bladder Strips | | |
|---|---|---|
| Compound | n | IC₅₀ (µM) |
| Example 2 | 4 | 2.0±1.0 |
| Example 4 | 4 | 9.8±5.1 |
| Example 6 | 2 | 0.11±0.002 |
| Example 7 | 2 | 15.8±0.05 |
| Example 8 | 2 1 | C^{b}=82.8±26.0% I^{a}=10.2% |
| Example 9 | 8 | 0.20±0.06 |
| Example 11 | 4 | 1.3±0.6 |
| Example 12 | 2 | 0.22±0.08 |
| Example 14 | 4 | I^{a}=22±5.9% |
| Example 15 | 6 | 2.0±0.8 |
| Example 16 | 2 4 | 9.35±0.46 I^{a}=36±3.2% |
| Example 17 | 2 | C^{b}=245±15% |
| Example 18 | 3 | I^{a}=26±13.8% |
| Example 19 | 2 | C^{b}=92.5±44.2% |
| Example 20 | 2 | 2.53±0.19 |
| Example 22 | 2 | 24.0±9.3 |
| Example 23 | 2 | 0.34±0.22 |
| Example 24 | 4 3 1 | 1.48±0.57 I^{a}=32.5±3.1% C^{b}=30% |
| Example 25 | 4 2 | 8.9±4.0 C^{b}=57.9±4.2% |

| | | |
|---|---|---|
| ^{a} Percent inhibition at 30 µM | | |
| ^{b} Percent contraction at 30 µM ^{c} A second lot (mp 215.5-216.0°C (softens 211.5°C)) exhibited an IC₅₀=0.13±0.03µM (n=6). | | |

In addition, we tested the ability of compounds to inhibit the hyperactivity of hypertrophied bladder (detrussor) smooth muscle in conscious female rats with hypertrophied bladders and thereby alleviate urinary incontinence in rats according to the following protocol described by Malmgren et al., J. Urol. 142:1134, 1989:

Female Sprague-Dawley rats, ranging in weight from 190-210 g are used. Up to 25 animals are prepared each time. After development of bladder hypertrophy 4-8 animals are used per test.

Compounds are dissolved in PEG-200 and administered by gastric gavage or intravenously in a volume of 5 ml/kg. For primary screening all drugs are administered at the arbitrary dose of 10 mg/kg p.o. to groups of 4 rats.

The animals are anesthetized with halothane. Through a midline incision the bladder and urethra are exposed and a ligature of 4-0 silk is tied around the proximal urethra in the presence of a stainless steel rod (1 mm diameter) to produce a partial occlusion. The rod is then removed. The abdominal region is closed using surgical staples and each rat receives 150,000 units of bicillin C-R. The animals are allowed six weeks to develop sufficient bladder hypertrophy. After six weeks, the ligature is removed under halothane anesthesia and a catheter (PE 60) with a cuff is placed in the dome of the bladder and secured with a purse string suture. The catheter is tunneled under the skin and exteriorized through an opening in the back of the neck. The abdominal incision is sutured and the free end of the catheter sealed. In order to prevent infections the rats receive an injection of bicillin C-R (150000 units/rat). Two days later the animals are used in cystometrical evaluations, The animals are placed in the metabolic cages and the catheter is attached (using a "T" connector) to a Statham pressure transducer (Model P23Db) and to a Harvard infusion pump. A plastic beaker attached to a force displacement transducer (Grass FTO3) is placed under the rat's cage to collect and record urine volume. Animals are allowed 15-30 minutes to rest before the saline infusion (20 ml/hr for 20 minutes) is started for the first cystometry period. Two hours after the first cystometry period, the rats are dosed with the vehicle or the test compound and one hour later a second cystometry is performed.

The following urodynamic variables are recorded:
- Basal bladder pressure =: the lowest bladder pressure during cystometry
- Threshold pressure =: bladder pressure immediately prior to micturition
- Micturition volume =: volume expelled
- Micturition pressure =: peak pressure during voiding
- Spontaneous activity =: mean amplitude of bladder pressure fluctuations during filling

### Presentation of results:

The mean value of each variable is calculated before and after compound administration. For each compound the changes in the variables measured are compared to the values obtained before treatment and expressed as percent inhibition. The data are also subjected to 2-way analysis of variance to determine significant (p<0.05) changes in the variable measured. The most characteristic finding in this rat model is spontaneous bladder contractions which develop during filling. The results of this study are shown in Table II.

**Table II**

| **Inhibition of Spontaneous Contractions In Vivo** | | | |
|---|---|---|---|
| Compound | # of animals | dose mg/kg (p.o.) | % Red (F)^{d} |
| Example 6 | 7 10 | 1 mg/kg 3 mg/kg | -40±13^{f} -58±9^{f} |
| Example 9 | 6 4 | 1 mg/kg 3 mg/kg | -63±13 -82±5 |
| Example 12 | 3 | 3 mg/kg | -53±18 |
| Example 17 | 2 | 10 mg/kg | -87±2 |

| | | | |
|---|---|---|---|
| ^{d} Percent reduction in the total number of spontaneous contractions in the hypertrophied rat bladder model | | | |
| ^{f} Findings obtained on a prior lot of this compound, mp. 195-196°C, IC₅₀=0.21±0.04 µM (n=4) | | | |

Hence, the compounds of this invention have a pronounced effect on smootl muscle contractility and are useful in the treatment of urinary incontinence, irritable bladder and bowel disease, asthma, hypertension, stroke, and similar diseases as mentioned above, which are amenable to treatment with potassium channel activating compounds by administration, orally parenterally, or by aspiration to a patient in neec thereof.

## Claims

1. A compound of the formula (I): 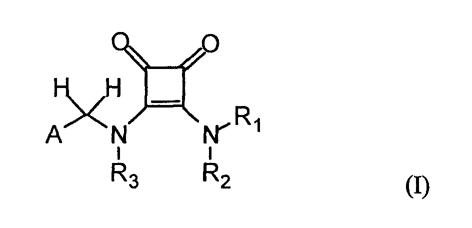 wherein:
R₁ is straight chain alkyl of 1 to 10 carbon atoms, branched chain alkyl of 3 to 10 carbon atoms, cycloalkyl of 3 to 10 carbon atoms, hydroxyalkyl of 2 to 10 carbon atoms or fluoroalkyl of 1 to 10 carbon atoms;
R₂ and R₃ are, independently, hydrogen or an acyl substituent selected from the group consisting of formyl, alkanoyl of 2 to 7 carbon atoms, alkenoyl of 3 to 7 carbon atoms, straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms, aralkoxycarbonyl of 6 to 12 carbon atoms, alkylsulfonyl of 1 to 7 carbon atoms, aroyl of 7 to 12 carbon atoms, arylalkenoyl of 9 to 20 carbon atoms, arylsulfonyl of 6 to 12 carbon atoms, arylalkanoyl of 8 to 12 carbon atoms or arylalkylsulfonyl of 7 to 12 carbon atoms; with the proviso that when R₃ is straight chain alkoxycarbonyl of 2 to 11 carbon atoms, branched chain alkoxycarbonyl of 4 to 11 carbon atoms, cycloalkoxycarbonyl of 4 to 11 carbon atoms, alkenoxycarbonyl of 2 to 11 carbon atoms or aralkoxycarbonyl of 6 to 12 carbon atoms, R₂ must be hydrogen;
A is a substituted phenyl group of the following formula: 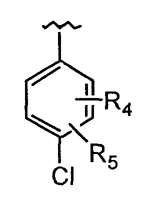 wherein:
R₄ and R₅ are, independently, cyano, nitro, amino, alkyl of 1 to 6 carbon atoms, fluoroalkyl of 1 to 6 carbon atoms, alkoxy of 1 to 6 carbon atoms, fluoroalkoxy of 1 to 6 carbon atoms, amino, alkylamino of 1 to 6 carbon atoms, dialkylamino of 2 to 12 carbon atoms, sulfamyl, alkylsulfonamido of 1 to 6 carbon atoms, arylsulfonamido of 6 to 12 carbon atoms, carbamoyl, alkylcarbamoyl of 2 to 7 carbon atoms, dialkylcarbamoyl of 4 to 14 carbon atoms, alkylcarboxamido containing 2 to 7 carbon atoms, arylcarboxamido containing 7 to 13 carbon atoms, alkylsulfonyl of 1 to 6 carbon atoms, perfluoroalkylsulfonyl of 1 to 6 carbon atoms, arylsulfonyl of 6 to 12 carbon atoms, chloro, bromo, fluoro, iodo, 1-imidazolyl, carboxyl or hydrogen, with the proviso that R₄ and R₅ cannot both be hydrogen;
or a pharmaceutically acceptable salt thereof.

2. A compound according to claim 1 in which R₁ is straight chain alkyl of 1 to 10 carbon atoms, branched chain alkyl of 3 to 10 carbon atoms or fluoroalkyl of 1 to 10 carbon atoms; or a pharmaceutically acceptable salt thereof.

3. A compound according to claim 1 or 2 in which R₁ is branched alkyl of 3 to 10 carbon atoms or polyfluoroalkyl of 1 to 10 carbon atoms, or a pharmaceutically acceptable salt thereof.

4. A compound according to any of the preceding claims in which R₂ and R₃ are, independently, hydrogen, alkanoyl of 2 to 7 carbon atoms, alkenoyl of 3 to 7 carbon atoms, aroyl of 7 to 12 carbon atoms, arylalkenoyl of 9 to 20 carbon atoms, straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms; with the proviso that when R₃ is straight chain alkoxycarbonyl of 2 to 7 carbon atoms, branched chain alkoxycarbonyl of 4 to 7 carbon atoms, alkenoxycarbonyl of 4 to 7 carbon atoms, or aralkoxycarbonyl of 6 to 12 carbon atoms, R₂ must be hydrogen; or a pharmaceutically acceptable salt thereof.

5. A compound according to any of the preceding claims in which R₂ and R₃ are, independently, hydrogen, alkanoyl of 2 to 7 carbon atoms, alkenoyl of 3 to 7 carbon atoms, straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms; with the proviso that when R₃ is straight chain alkoxycarbonyl of 3 or 5 carbon atoms, branched chain alkoxycarbonyl of 5 carbon atoms, alkenoxycarbonyl of 4 carbon atoms, or aralkoxycarbonyl of 8 carbon atoms, R₂ must be hydrogen, or a pharmaceutically acceptable salt thereof.

6. A compound according to any of the preceding claims in which R₂ and R₃ are, independently, hydrogen, alkanoyl of 2 to 7 carbon atoms, alkenoyl of 3 to 7 carbon atoms, aroyl of 7 to 12 carbon atoms, or arylalkenoyl of 9 to 20 carbon atoms;
or a pharmaceutically acceptable salt thereof.

7. A compound according to any of the preceding claims in which R₂ and R₃ are, independently, hydrogen, alkanoyl of 2 to 7 carbon atoms or alkenoyl of 3 to 7 carbon atoms; or a pharmaceutically acceptable salt thereof.

8. A compound according to any of claims 1 to 7 in which R₄ and R₅ are, independently, cyano, alkyl of 1 to 6 carbon atoms, fluoroalkyl of 1 to 6 carbon atoms, alkoxy of 1 to 6 carbon atoms, fluoroalkoxy of 1 to 6 carbon atoms, chloro, bromo, fluoro, iodo or hydrogen, with the proviso that R₄ and R₅ cannot both be hydrogen; or a pharmaceutically acceptable salt thereof.

9. A compound according to claim 8 wherein R₄ and R₅ are, independently, cyano, methyl, ethyl, trifluoromethyl, fluoroalkyl of 1 to 2 carbon atoms, methoxy, ethoxy, trifluoromethoxy, fluoroalkoxy of 1 to 2 carbon atoms, chloro, bromo, fluoro or hydrogen, with the proviso that R₄ and R₅ cannot both be hydrogen; or a pharmaceutically acceptable salt thereof.

10. The compound of Claim 1 which is:
3-(2,4-dichlorobenzylamino)-4-(1,2,2-trimethylpropylamino)-cyclobut-3-ene-1,2-dione;
3-(2,4-dichloro-6-methyl-benzylamino)-4-(1,2,2-trimethyl-propylamino)-cyclobut-3-ene-1,2-dione;
3-(2,4-dichlorobenzylamino)-4-(1,1-dimethylpropylamino)-cyclobut-3-ene-1,2-dione;
N-(2,4-dichlorobenzyl)-N-[2-(1,1-dimethyl-propylamino)-3,4-dioxo-cyclobut-1-enyl]-acetamide;
N-(2,4-dichlorobenzyl)-N-[2-(1,1-dimethyl-propylamino)-3,4-dioxo-cyclobut-1-enyl]-butyramide;
3 -(2,4-dichloro-6-methyl-benzylamino)-4-(1,1-dimethyl-propylamino)-cyclobut-3-ene-1,2-dione;
3-(t-butylamino)-4-(2,4-dichlorobenzylamino)-cyclobut-3-ene-1,2-dione;
3-tert-butylamino-4-(2,4-dichloro-6-methyl-benzylamino)-cyclobut-3-ene-1,2-dione;
3-(3,4-dichlorobenzylamino)-4-(1-ethyl-propylamino)-cyclobut-3-ene-1,2-dione;
3-(2,4-dichlorobenzylamino)-4-(1-ethyl-propylamino)-cyclobut-3-ene-1,2-dione;
3-(2,4-dichloro-6-methyl-benzylamino)-4-(2,2,3,3,3-pentafluoropropylamino)-cyclobut-3-ene-1,2-dione;
N-(2,4-dichloro-6-methyl-benzyl)-N-[2-(1,1-dimethyl-propylamino)-3,4-dioxo-cyclobut-1-enyl]-butyramide;
N-(2,4-dichloro-6-methyl-benzyl)-N-[2-(1,1-dimethyl-propylamino)-3,4-dioxo-cyclobut-1-enyl]-acetamide;
N-(tert-butyl)-N-[2-(2,4-dichloro-6-methyl-benzylamino)-3,4-dioxo-cyclobut-1-enyl]-propionamide;
3-(2,4-dichloro-6-methyl-benzylamino)-4-(1,2-dimethyl-2-fluoropropylamino)-cyclobut-3-ene-1,2-dione;
3-(2,4-dichlorobenzylamino)-4-(2-hydroxy-1,1-dimethylethylamino)-cyclobut-3-ene-1,2-dione;
(R)-3-(2,4-dichlorobenzylamino)-4-(1,2,2-trimethylpropylamino)-cyclobut-3-ene-1,2-dione;
3 -tert-butylamino-4-(3,4-dichlorobenzylamino)-cyclobut-3-ene-1,2-dione;
3-(3,4-dichlorobenzylamino)-4-(1,1-dimethylpropylamino)-cyclobut-3-ene-1,2-dione;
(2,4-dichloro-6-methyl-benzyl)-[2-(1,1-dimethyl-propylamino)-3,4-dioxocyclobut-1-enyl]-carbamic acid tert-butyl ester;
or a pharmaceutically acceptable salts thereof.

11. A pharmaceutical composition of matter comprising a compound of formula (I) as defined in any of claims 1 to 10 or a pharmaceutically acceptable salt thereof and a pharmaceutically acceptable carrier therefor.

12. A compound as claimed in any of claims 1 to 10 for use as a pharmaceutical or therapeutic substance.

13. A compound as claimed in any of claims 1 to 10 for use in the treatment of diseases or conditions related to smooth muscle contractions

14. A compound according to claim 13 in which the smooth muscle adversely contracting causes urinary incontinence.

15. A compound according to claim 13 in which the smooth muscle adversely contracting causes irritable bowel syndrome.

16. A process for the preparation of the compounds of formula I as defined in any of claims 1 to 10 which comprises reacting a compound of formula (II): 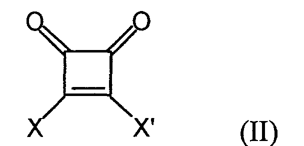 wherein X and X' are leaving groups with a compound of formula (III):
A₁―CH₂NH₂ (III)
wherein A₁ is A as defined hereinbefore or a group of atoms convertible thereto,
followed by treatment with a compound of formula (IV): 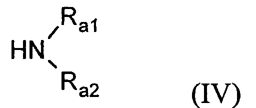 wherein Rₐ₁ and Rₐ₂ are R₁ and R₂, respectively, as defined hereinbefore or a group of atoms convertible thereto in a solvent, and optionally reacting the product with an appropriate anhydride in pyridine with or without protection of the benzyl nitrogen thereby attaching R₂ and/or R₃, and optionally thereafter forming a pharmaceutically acceptable salt thereof.

17. Process according to claim 16 in which the solvent is ethanol, acetonitrile or the appropriate amine (IV)

18. Process according to claim 16 or 17 in which dichloromethane is used as a cosolvent.

19. Process according to any of claims 16 to 18 which comprises reacting a sodium, potassium, or lithium salt of compound of formula (II), where X is methoxy, ethoxy, butoxy, isopropoxy, or similar leaving group and X' is NHR₁, with the appropriate anhydride in dichloromethane, tetrahydrofuran and/or N,N-dimethylformamide or any other suitable solvent, followed by treatment with a compound of formula (III) as defined above in a solvent such as acetonitrile at room temperature.

## Patentansprüche

1. Verbindung der Formel (I), 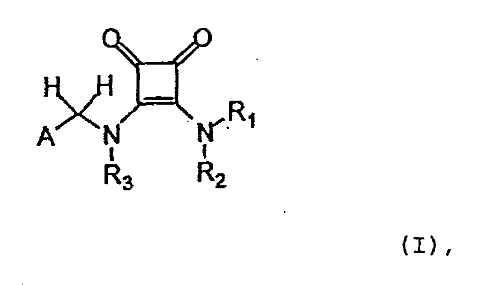 worin:
R₁ gradkettiges Alkyl mit 1 bis 10 Kohlenstoffatomen, verzweigtkettiges Alkyl mit 3 bis 10 Kohlenstoffatomen, Cycloalkyl mit 3 bis 10 Kohlenstoffatomen, Hydroxyalkyl mit 2 bis 10 Kohlenstoffatomen oder Fluoralkyl mit 1 bis 10 Kohlenstoffatomen darstellt.
R₂ und R₃ unabhängig Wasserstoff oder einen Acylsubstituenten, ausgewählt aus der Gruppe bestehend aus Formyl, Alkanoyl mit 2 bis 7 Kohlenstoffatomen, Alkenoyl mit 3 bis 7 Kohlenstoffatomen, gradkettigem Alkoxycarbonyl mit 2 bis 11 Kohlenstoffatomen, verzweigtkettigem Alkoxycarbonyl mit 4 bis 11 Kohlenstoffatomen, Cycloalkoxycarbonyl mit 4 bis 11 Kohlenstoffatomen, Alkenoxycarbonyl mit 2 bis 11 Kohlenstoffatomen, Aralkoxycarbonyl mit 6 bis 12 Kohlenstoffatomen, Alkylsulfonyl mit 1 bis 7 Kohlenstoffatomen, Aroyl mit 7 bis 12 Kohlenstoffatomen, Arylalkenoyl mit 9 bis 20 Kohlenstoffatomen, Arylsulfonyl mit 6 bis 12 Kohlenstoffatomen, Arylalkanoyl mit 8 bis 12 Kohlenstoffatomen oder Arylalkylsulfonyl mit 7 bis 12 Kohlenstoffatomen darstellen; unter der Bedingung, dass wenn R₃ gradkettiges Alkoxycarbonyl mit 2 bis 11 Kohlenstoffatomen, verzweigtkettiges Alkoxycarbonyl mit 4 bis 11 Kohlenstoffatomen, Cycloalkoxycarbonyl mit 4 bis 11 Kohlenstoffatomen, Alkenoxycarbonyl mit 2 bis 11 Kohlenstoffatomen oder Aralkoxycarbonyl mit 6 bis 12 Kohlenstoffatomen darstellt, R₂ für Wasserstoff stehen muss;
A eine substituierte Phenylgruppe der folgenden Formel darstellt: 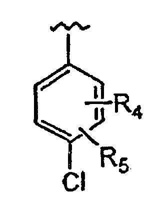 worin:
R₄ und R₅ unabhängig Cyano, Nitro, Amino, Alkyl mit 1 bis 6 Kohlenstoffatomen, Fluoralkyl mit 1 bis 6 Kohlenstoffatomen, Alkoxy mit 1 bis 6 Kohlenstoffatomen, Fluoralkoxy mit 1 bis 6 Kohlenstoffatomen, Alkylamino mit 1 bis 6 Kohlenstoffatomen, Dialkylamino mit 2 bis 12 Kohlenstoffatomen, Sulfamyl, Alkylsulfonamido mit 1 bis 6 Kohlenstoffatomen, Arylsulfonamido mit 6 bis 12 Kohlenstoffatomen, Carbamoyl, Alkylcarbamoyl mit 2 bis 7 Kohlenstoffatomen, Dialkylcarbamoyl mit 4 bis 14 Kohlenstoffatomen, Alkylcarboxamido, enthaltend 2 bis 7 Kohlenstoffatome, Arylcarboxamido, enthaltend 7 bis 13 Kohlenstoffatome, Alkylsulfonyl mit 1 bis 6 Kohlenstoffatomen, Perfluoralkylsulfonyl mit 1 bis 6 Kohlenstoffatomen, Arylsulfonyl mit 6 bis 12 Kohlenstoffatomen, Chlor, Brom, Fluor, Iod, 1-Imidazolyl, Carboxyl oder Wasserstoff darstellen; unter der Bedingung, dass R₄ und R₅ nicht beide Wasserstoff darstellen können;
oder ein pharmazeutisch annehmbares Salz davon.

2. Verbindung gemäß Anspruch 1, worin R₁ ein gradkettiges Alkyl mit 1 bis 10 Kohlenstoffatomen, verzweigtkettiges Alkyl mit 3 bis 10 Kohlenstoffatomen oder Fluoralkyl mit 1 bis 10 Kohlenstoffatomen darstellt; oder ein pharmazeutisch annehmbares Salz davon.

3. Verbindung gemäß Anspruch 1 oder 2, worin R₁ ein verzweigtkettiges Alkyl mit 3 bis 10 Kohlenstoffatomen oder Polyfluoralkyl mit 1 bis 10 Kohlenstoffatomen darstellt, oder ein pharmazeutisch annehmbares Salz davon.

4. Verbindung gemäß einem der vorhergehenden Ansprüche, worin R₂ und R₃ unabhängig Wasserstoff, Alkanoyl mit 2 bis 7 Kohlenstoffatomen, Alkenoyl mit 3 bis 7 Kohlenstoffatomen, Aroyl mit 7 bis 12 Kohlenstoffatomen, Arylalkenoyl mit 9 bis 20 Kohlenstoffatomen, gradkettiges Alkoxycarbonyl mit 2 bis 7 Kohlenstoffatomen, verzweigtkettiges Alkoxycarbonyl mit 4 bis 7 Kohlenstoffatomen, Alkenoxycarbonyl mit 4 bis 7 Kohlenstoffatomen oder Aralkoxycarbonyl mit 6 bis 12 Kohlenstoffatomen darstellt; unter der Bedingung, dass wenn R₃ gradkettiges Alkoxycarbonyl mit 2 bis 7 Kohlenstoffatomen, verzweigtkettiges Alkoxycarbonyl mit 4 bis 7 Kohlenstoffatomen, Alkenoxycarbonyl mit 4 bis 7 Kohlenstoffatomen oder Aralkoxycarbonyl mit 6 bis 12 Kohlenstoffatomen darstellt, R₂ Wasserstoff darstellen muss; oder ein pharmazeutisch annehmbares Salz davon.

5. Verbindung gemäß einem der vorhergehenden Ansprüche, worin R₂ und R₃ unabhängig Wasserstoff, Alkanoyl mit 2 bis 7 Kohlenstoffatomen, Alkenoyl mit 3 bis 7 Kohlenstoffatomen, gradkettigem Alkoxycarbonyl mit 3 oder 5 Kohlenstoffatomen, verzweigtkettigem Alkoxycarbonyl mit 5 Kohlenstoffatomen, Alkenoxycarbonyl mit 4 Kohlenstoffatomen oder Aralkoxycarbonyl mit 8 Kohlenstoffatomen darstellen; unter der Bedingung, dass wenn R₃ gradkettiges Alkoxycarbonyl mit 3 oder 5 Kohlenstoffatomen, verzweigtkettiges Alkoxycarbonyl mit 5 Kohlenstoffatomen, Alkenoxycarbonyl mit 4 Kohlenstoffatomen oder Aralkoxycarbonyl mit 8 Kohlenstoffatomen darstellt, R₂ Wasserstoff darstellen muss; oder ein pharmazeutisch annehmbares Salz davon.

6. Verbindung gemäß einem der vorhergehenden Ansprüche, worin R₂ und R₃ unabhängig Wasserstoff, Alkanoyl mit 2 bis 7 Kohlenstoffatomen, Alkenoyl mit 3 bis 7 Kohlenstoffatomen, Aroyl mit 7 bis 12 Kohlenstoffatomen oder Arylalkenoyl mit 9 bis 20 Kohlenstoffatomen darstellen;
oder ein pharmazeutisch annehmbares Salz davon.

7. Verbindung gemäß einem der vorhergehenden Ansprüche, worin R₂ und R₃ unabhängig Wasserstoff, Alkanoyl mit 2 bis 7 Kohlenstoffatomen oder Alkenoyl mit 3 bis 7 Kohlenstoffatomen darstellen; oder ein pharmazeutisch annehmbares Salz davon.

8. Verbindung gemäß einem der Ansprüche 1 bis 7, worin R₄ und R₅ unabhängig Cyano, Alkyl mit 1 bis 6 Kohlenstoffatomen, Fluoralkyl mit 1 bis 6 Kohlenstoffatomen, Alkoxy mit 1 bis 6 Kohlenstoffatomen, Fluoralkoxy mit 1 bis 6 Kohlenstoffatomen, Chlor, Brom, Fluor, Iod oder Wasserstoff darstellen, unter der Bedingung, dass R₄ und R₅ nicht beide Wasserstoff darstellen können; oder ein pharmazeutisch annehmbares Salz davon.

9. Verbindung gemäß Anspruch 8, worin R₄ und R₅ unabhängig Cyano, Methyl, Ethyl, Trifluormethyl, Fluoralkyl mit 1 bis 2 Kohlenstoffatomen, Methoxy, Ethoxy, Trifluormethoxy, Fluoralkoxy mit 1 bis 2 Kohlenstoffatomen, Chlor, Brom, Fluor oder Wasserstoff darstellen, unter der Bedingung, dass R₄ und R₅ nicht beide Wasserstoff darstellen können; oder ein pharmazeutisch annehmbares Salz davon.

10. Verbindung nach Anspruch 1, welche ist:
3-(2,4-Dichlorbenzylamino)-4-(1,2,2-trimethylpropylamino)-cyclobut-3-en-1,2-dion;
3-(2,4-Dichlor-6-methyl-benzylamino)-4-(1,2,2-trimethylpropylamino)-cyclobut-3-en-1,2-dion;
3-(2,4-Dichlorbenzylamino)-4-(1,1-dimethylpropylamino)-cyclobut-3-en-1,2-dion;
N-(2,4-Dichlorbenzyl)-N-[2-(1,1-dimethyl-propylamino)-3,4-dioxo-cyclobut-1-enyl]-acetamid;
N-(2,4-Dichlorbenzyl)-N-[2-(1,1-dimethyl-propylamino)-3,4-dioxo-cyclobut-1-enyl]-butyramid;
3-(2,4-Dichlor-6-methyl-benzylamino)-4-(1,1-dimethylpropylamino)-cyclobut-3-en-1,2-dion;
3-(t-Butylamino)-4-(2,4-dichlorbenzylamino)-cyclobut-3-en-1,2-dione;
3-tert-Butylamino-4-(2,4-dichlor-6-methyl-benzylamino)-cyclobut-3-en-1,2-dion;
3-(3,4-Dichlorbenzylamino)-4-(1-ethyl-propylamino)-cyclobut-3-en-1,2-dion;
3-(2,4-Dichlorobenzylamino)-4-(1-ethyl-propylamino)-cyclobut-3-en-1,2-dion;
3-(2,4-Dichlor-6-methyl-benzylamino)-4-(2,2,3,3,3-pentafluor-propylamino)-cyclobut-3-en-1,2-dion;
N-(2,4-Dichlor-6-methyl-benzyl)-N-[2-(1,1-dimethylpropylamino)-3, 4-dioxo-cyclobut-1-enyl]-butyramid;
N-(2,4-Dichlor-6-methyl-benzyl)-N-[2-(1,1-dimethylpropylamino)-3,4-dioxo-cyclobut-1-enyl]-acetamid;
N-(tert-Butyl)-N-[2-(2,4-dichlor-6-methyl-benzylamino)-3,4-dioxo-cyclobut-1-enyl]-propionamid;
3-(2,4-Dichlor-6-methyl-benzylamino)-4-(1,2-dimethyl-2-fluor-propylamino)-cyclobut-3-en-1,2-dion;
3-(2,4-Dichlorbenzylamino)-4-(2-hydroxy-1,1-dimethylethylamino)-cyclobut-3-en-1,2-dion;
(R)-3-(2,4-Dichlorbenzylamino)-4-(1,2,2-trimethylpropylamino) -cyclobut-3-en-1,2-dion;
3-tert-Butylamino-4-(3,4-dichlorbenzylamino)-cyclobut-3-en-1,2-dion;
3-(3,4-Dichlorbenzylamino)-4-(1,1-dimethylpropylamino)-cyclobut-3-en-1,2-dion;
(2,4-Dichlor-6-methyl-benzyl)-[2-(1,1-dimethyl-propylamino)-3,4-dioxo-cyclobut-1-enyl]-carbaminsäure tert-Butylester; oder ein pharmazeutisch annehmbares Salz davon.

11. Pharmazeutische Zusammensetzung aus Substanzen, welche eine Verbindung der Formel (I), wie in einem der Ansprüche 1 bis 10 definiert, oder ein pharmazeutisch annehmbares Salz davon, und einen pharmazeutisch annehmbaren Träger dafür umfasst.

12. Verbindung, wie in einem der Ansprüche 1 bis 10 beansprucht, zur Verwendung als pharmazeutische oder therapeutische Substanz.

13. Verbindung, wie in einem der Ansprüche 1 bis 10 beansprucht, zur Verwendung bei der Behandlung von Erkrankungen oder Zuständen in Verbindung mit Glattmuskelkontraktionen.

14. Verbindung gemäß Anspruch 13, wobei das ungünstige Kontrahieren des Glattmuskels Harninkontinenz verursacht.

15. Verbindung gemäß Anspruch 13, wobei das ungünstige Kontrahieren des Glattmuskels Reizdarmsyndrom verursacht.

16. Verfahren zur Herstellung der Verbindungen der Formel I, wie in einem der Ansprüche 1 bis 10 definiert, welches umfasst:
Umsetzen einer Verbindung der Formel (II): 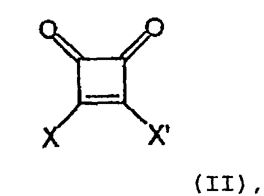 worin X und X' Abgangsgruppen darstellen, mit einer Verbindung der Formel (III):
A₁―CH₂NH₂ (III)
worin A₁ für A steht, wie hierin vorher definiert, oder eine dazu konvertierbare Gruppe von Atomen,
gefolgt von Behandlung mit einer Verbindung der Formel (IV) : 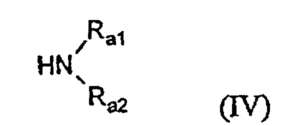 worin Rₐ₁ und Rₐ₂ jeweils R₁ und R₂ darstellen, wie hierin vorher definiert, oder eine dazu konvertierbare Gruppe von Atomen, in einem Lösungsmittel, und gegebenenfalls Umsetzen des Produkts mit einem passenden Anhydrid in Pyridin mit oder ohne Schutz des Benzylstickstoffs, dadurch Anbinden von R₂ und/oder R₃ und gegebenenfalls danach Bilden eines pharmazeutisch annehmbaren Salzes davon.

17. Verfahren gemäß Anspruch 16, wobei das Lösungsmittel Ethanol, Acetonitril oder das passende Amin (IV) ist.

18. Verfahren gemäß Anspruch 16 oder 17, wobei Dichlormethan als Co-Lösungsmittel verwendet wird.

19. Verfahren gemäß einem der Ansprüche 16 bis 18, welches umfasst: Umsetzen eines Natrium-, Kalium- oder Lithiumsalzes von Verbindung der Formel (II), worin X für Methoxy, Ethoxy, Butoxy, Isopropoxy oder eine ähnliche Abgangsgruppe steht, und X' für NHR₁ steht, mit dem passenden Anhydrid in Dichlormethan, Tetrahydrofuran und/oder N,N-Dimethylformamid oder jedem anderen geeigneten Lösungsmittel, gefolgt von Behandlung mit einer Verbindung der Formel (III), wie oben definiert, in einem Lösungsmittel wie Acetonitril bei Raumtemperatur.

## Revendications

1. Composé de formule (I) 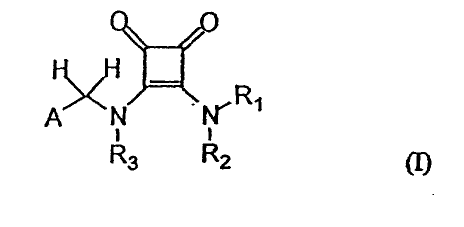 dans laquelle :
R₁ est alkyle à chaîne droite de 1 à 10 atomes de carbone, alkyle à chaîne ramifiée de 3 à 10 atomes de carbone, cycloalkyle de 3 à 10 atomes de carbone, hydroxyalkyle de 2 à 10 atomes de carbone ou fluoroalkyle de 1 à 10 atomes de carbone ;
R₂ et R₃ sont, indépendamment, hydrogène ou un substituant acyle choisi parmi le groupe constitué du formyle, d'alcanoyle de 2 à 7 atomes de carbone, d'alcénoyle de 3 à 7 atomes de carbone, d'alkoxycarbonyle à chaîne droite de 2 à 11 atomes de carbone, d'alkoxycarbonyle à chaîne ramifiée de 4 à 11 atomes de carbone, de cycloalkoxycarbonyle de 4 à 11 atomes de carbone, d'alcénoxycarbonyle de 2 à 11 atomes de carbone, d'aralkoxycarbonyle de 6 à 12 atomes de carbone, d'alkylsulfonyle de 1 à 7 atomes de carbone, d'aroyle de 7 à 12 atomes de carbone, d'arylalcénoyle de 9 à 20 atomes de carbone, d'arylsulfonyle de 6 à 12 atomes de carbone, d'arylalcanoyle de 8 à 12 atomes de carbone ou d'arylalkylsulfonyle de 7 à 12 atomes de carbone ; à condition que, quand R₃ est alkoxycarbonyle à chaîne droite de 2 à 11 atomes de carbone, alkoxycarbonyle à chaîne ramifiée de 4 à 11 atomes de carbone, cycloalkoxycarbonyle de 4 à 11 atomes de carbone, alcénoxycarbonyle de 2 à 11 atomes de carbone ou aralkoxycarbonyle de 6 à 12 atomes de carbone, R₂ doit être hydrogène ;
A est un groupement phényle substitué de formule suivante : 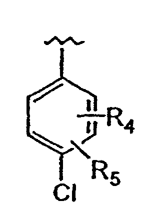 dans laquelle :
R₄ et R₅ sont, indépendamment, cyano, nitro, amino, alkyle de 1 à 6 atomes de carbone, fluoroalkyle de 1 à 6 atomes de carbone, alkoxy de 1 à 6 atomes de carbone, fluoroalkoxy de 1 à 6 atomes de carbone, amino, alkylamino de 1 à 6 atomes de carbone, dialkylamino de 2 à 12 atomes de carbone, sulfamyle, alkylsulfonamido de 1 à 6 atomes de carbone, arylsulfonamido de 6 à 12 atomes de carbone, carbamoyle, alkylcarbamoyle de 2 à 7 atomes de carbone, dialkylcarbamoyle de 4 à 14 atomes de carbone, alkylcarboxamido contenant 2 à 7 atomes de carbone, arylcarboxamido contenant 7 à 13 atomes de carbone, alkylsulfonyle de 1 à 6 atomes de carbone, perfluoroalkylsulfonyle de 1 à 6 atomes de carbone, arylsulfonyle de 6 à 12 atomes de carbone, chloro, bromo, fluoro, iodo, 1-imidazolyle, carboxyle ou hydrogène, à condition que R₄ et R₅ ne peuvent pas être tous les deux hydrogène ;
ou un sel pharmaceutiquement acceptable de celui-ci.

2. Composé selon la revendication 1 dans lequel R₁ est alkyle à chaîne droite de 1 à 10 atomes de carbone, alkyle à chaîne ramifiée de 3 à 10 atomes de carbone ou fluoroalkyle de 1 à 10 atomes de carbone ; ou un sel pharmaceutiquement acceptable de celui-ci.

3. Composé selon la revendication 1 ou 2 dans lequel R₁ est alkyle ramifié de 3 à 10 atomes de carbone ou polyfluoroalkyle de 1 à 10 atomes de carbone, ou un sel pharmaceutiquement acceptable de celui-ci.

4. Composé selon l'une quelconque des revendications précédentes dans lequel R₂ et R₃ sont, indépendamment, hydrogène, alcanoyle de 2 à 7 atomes de carbone, alcénoyle de 3 à 7 atomes de carbone, aroyle de 7 à 12 atomes de carbone, arylalcénoyle de 9 à 20 atomes de carbone, alkoxycarbonyle à chaîne droite de 2 à 7 atomes de carbone, alkoxycarbonyle à chaîne ramifiée de 4 à 7 atomes de carbone, alcénoxycarbonyle de 4 à 7 atomes de carbone ou aralkoxycarbonyle de 6 à 12 atomes de carbone ; à condition que, quand R₃ est alkoxycarbonyle à chaîne droite de 2 à 7 atomes de carbone, alkoxycarbonyle à chaîne ramifiée de 4 à 7 atomes de carbone, alcénoxycarbonyle de 4 à 7 atomes de carbone, ou aralkoxycarbonyle de 6 à 12 atomes de carbone, R₂ doit être hydrogène ; ou un sel pharmaceutiquement acceptable de celui-ci.

5. Composé selon l'une quelconque des revendications précédentes dans lequel R₂ et R₃ sont, indépendamment, hydrogène, alcanoyle de 2 à 7 atomes de carbone, alcénoyle de 3 à 7 atomes de carbone, alkoxycarbonyle à chaîne droite de 3 ou 5 atomes de carbone, alkoxycarbonyle à chaîne ramifiée de 5 atomes de carbone, alcénoxycarbonyle de 4 atomes de carbone ou aralkoxycarbonyle de 8 atomes de carbone ; à condition que quand R₃ est alkoxycarbonyle à chaîne droite de 3 ou 5 atomes de carbone, alkoxycarbonyle à chaîne ramifiée de 5 atomes de carbone, alcénoxycarbonyle de 4 atomes de carbone ou aralkoxycarbonyle de 8 atomes de carbone, R₂ doit être hydrogène ou un sel pharmaceutiquement acceptable de celui-ci.

6. Composé selon l'une quelconque des revendications précédentes dans lequel R₂ et R₃ sont, indépendamment, hydrogène, alcanoyle de 2 à 7 atomes de carbone, alcénoyle de 3 à 7 atomes de carbone, aroyle de 7 à 12 atomes de carbone ou arylalcénoyle de 9 à 20 atomes de carbone ;
ou un sel pharmaceutiquement acceptable de celui-ci.

7. Composé selon l'une quelconque des revendications précédentes dans lequel R₂ et R₃ sont, indépendamment, hydrogène, alcanoyle de 2 à 7 atomes de carbone ou alcénoyle de 3 à 7 atomes de carbone ; ou un sel pharmaceutiquement acceptable de celui-ci.

8. Composé selon l'une quelconque des revendications 1 à 7 dans lequel R₄ et R₅ sont, indépendamment, cyano, alkyle de 1 à 6 atomes de carbone, fluoroalkyle de 1 à 6 atomes de carbone, alkoxy de 1 à 6 atomes de carbone, fluoroalkoxy de 1 à 6 atomes de carbone, chloro, bromo, fluoro, iodo ou hydrogène, à condition que R₄ et R₅ ne peuvent pas être tous les deux hydrogène ; ou un sel pharmaceutiquement acceptable de celui-ci.

9. Composé selon la revendication 8 dans lequel R₄ et R₅ sont, indépendamment, cyano, méthyle, éthyle, trifluorométhyle, fluoroalkyle de 1 à 2 atomes de carbone, méthoxy, éthoxy, trifluorométhoxy, fluoroalkoxy de 1 à 2 atomes de carbone, chloro, bromo, fluoro ou hydrogène, à condition que R₄ et R₅ ne peuvent pas être tous deux hydrogène ; ou un sel pharmaceutiquement acceptable de celui-ci.

10. Composé selon la revendication 1 qui est :
la 3-(2,4-dichlorobenzylamino)-4-(1,2,2-triméthylpropylamino)-cyclobut-3-ène-1,2-dione ;
la 3-(2,4-dichloro-6-méthylbenzylamino)-4-(1,2,2-triméthylpropylamino)-cyclobut-3-ène-1,2-dione ;
la 3-(2,4-dichlorobenzylamino)-4-(1,1-diméthylpropylamino)-cyclobut-3-ène-1,2-dione;
le N-(2,4-dichlorobenzyl)-N-[2-(1,1-diméthylpropylamino)-3,4-dioxo-cyclobut-1-ényl]-acétamide ;
le N-(2,4-dichlorobenzyl)-N-[2-(1,1-diméthylpropylamino)-3,4-dioxo-cyclobut-1-ényl]-butyramide ;
la 3-(2,4-dichloro-6-méthylbenzylamino)-4-(1,1-diméthylpropylamino)-cyclobut-3-ène-1,2-dione ;
la 3-(t-butylamino)-4-(2,4-dichlorobenzylamino)-cyclobut-3-ène-1,2-dione ;
la 3-(t-butylamino)-4-(2,4-dichloro-6-méthylbenzylamino)-cyclobut-3-ène-1,2-dione;
la 3-(3,4-dichlorobenzylamino)-4-(1-éthylpropylamino)-cyclobut-3-ène-1,2-dione;
la 3-(2,4-dichlorobenzylamino)-4-(1-éthylpropylamino)-cyclobut-3-ène-1,2-dione;
la 3-(2,4-dichloro-6-méthylbenzylamino)-4-(2,2,3,3,3-pentafluoropropylamino)-cyclobut-3-ène-1,2-dione ;
le N-(2,4-dichloro-6-méthylbenzyl)-N-[2-(1,1-diméthylpropylamino)-3,4-dioxo-cyclobut-1-ényl]-butyramide ;
le N-(2,4-dichloro-6-méthylbenzyl)-N-[2-(1,1-diméthylpropylamino)-3,4-dioxo-cyclobut-1-ényl]-acétamide ;
le N-(t-butyl)-N-[2-(2,4-dichloro-6-méthylbenzylamino)-3,4-dioxo-cyclobut-1-ényl]-propionamide ;
la 3-(2,4-dichloro-6-méthylbenzylamino)-4-(1,2-diméthyl-2-fluoropropylamino)-cyclobut-3-ène-1,2-dione ;
la 3-(2,4-dichlorobenzylamino)-4-(2-hydroxy-1,1-diméthyléthylamino)-cyclobut-3-ène-1,2-dione ;
la (R)-3-(2,4-dichlorobenzylamino)-4-(1,2,2-triméthylpropylamino)-cyclobut-3-ène-1,2-dione ;
la 3-t-buylamino-4-(3,4-dichlorobenzylamino)-cyclobut-3-ène-1,2-dione ;
la 3-(3,4-dichlorobenzylamino)-4-(1,1-diméthylpropylamino)-cyclobut-3-ène-1,2-dione;
l'ester t-butylique de l'acide (2,4-dichloro-6-méthylbenzylamino) - [2- (1,1-diméthylpropylamino)-3,4-dioxo-cyclobut-1-ényl]carbamique ;
ou un sel pharmaceutiquement acceptable de celui-ci.

11. Composition pharmaceutique de matière comprenant un composé de formule (I) comme défini dans l'une quelconque des revendications 1 à 10 ou un sel pharmaceutiquement acceptable de celui-ci et un support pharmaceutiquement acceptable.

12. Composé selon l'une quelconque des revendications 1 à 10 pour l'utilisation comme substance pharmaceutique ou thérapeutique.

13. Composé selon l'une quelconque des revendications 1 à 10 pour l'utilisation dans le traitement de maladies ou d'états liés à des contractions de muscles lisses.

14. Composé selon la revendication 13 dans lequel la contraction défavorable du muscle lisse provoque l'incontinence urinaire.

15. Composé selon la revendication 13 dans lequel la contraction défavorable du muscle lisse provoque le syndrome du côlon irritable.

16. Procédé de préparation des composés de formule (I) comme définis dans l'une quelconque des revendications 1 à 10 qui comprend la réaction d'un composé de formule (II) 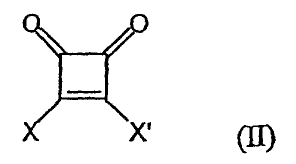 dans laquelle X et X' sont des groupements partants avec un composé de formule (III)
A₁-CH₂NH₂ (III)
dans laquelle A₁ est A comme défini ci-avant ou un groupement d'atomes convertibles en cela,
suivie par le traitement avec un composé de formule (IV) 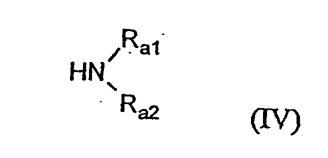 dans laquelle Rₐ₁ et Rₐ₂ sont R₁ et R₂, respectivement, comme définis ci-avant ou un groupement d'atomes convertibles en cela dans un solvant, et facultativement la réaction du produit avec un anhydride approprié dans la pyridine avec ou sans protection de l'azote du benzyle en attachant de cette façon R₂ et/ou R₃ et la formation facultative ultérieure d'un sel pharmaceutiquement acceptable de celui-ci.

17. Procédé selon la revendication 16 dans lequel le solvant est l'éthanol, l'acétonitril ou l'amine appropriée (IV).

18. Procédé selon la revendication 16 ou 17 dans lequel le dichlorométhane est utilisé comme cosolvant.

19. Procédé selon l'une quelconque des revendications 16 à 18 qui comprend la réaction d'un sel de sodium, potassium ou lithium du composé de formule (II), où X est méthoxy, éthoxy, butoxy, isopropoxy ou un groupement partant similaire et X' est NHR₁, avec l'anhydride approprié dans le dichlorométhane, le tétrahydrofuranne et/ou le N,N-diméthylformamide ou tout autre solvant approprié, suivie par le traitement avec un composé de formule (III) comme défini ci-dessus dans un solvant comme l'acétonitrile à température ambiante.
